# EUROPÄISCHE PATENTSCHRIFT

(11) **EP 3 851 063 B1**
(45) Veröffentlichungstag und Bekanntmachung des Hinweises auf die Patenterteilung: **12.07.2023**
(21) Anmeldenummer: 21150921.1
(22) Anmeldetag: 11.01.2021
(51) Int. Cl.: A61B 90/20, G02B 21/00, G02B 21/22, A61B 1/00, G02B 21/06, G02B 21/16

(54) **OPTISCHES BEOBACHTUNGSINSTRUMENT SOWIE VERFAHREN ZUM ERZEUGEN EINES STEREOBILDS EINES OBJEKTFELDS**
OPTICAL OBSERVATION INSTRUMENT AND METHOD FOR GENERATING A STEREO IMAGE OF AN OBJECT FIELD
INSTRUMENT OPTIQUE D'OBSERVATION AINSI QUE PROCÉDÉ DE GÉNÉRATION D'UNE IMAGE STÉRÉO D'UN CHAMP D'OBJET

(30) Priorität: 14.01.2020 DE 102020100674
(43) Veröffentlichungstag der Anmeldung: 21.07.2021
(73) Patentinhaber: KARL STORZ SE & Co. KG, 78532 Baden-Württemberg Tuttlingen (DE)
(72) Erfinder: Forster, Jonas, 78532 Tuttlingen (DE); Heni, Andreas, 78532 Tuttlingen (DE); Köhler, Benedikt, 78532 Tuttlingen (DE); Vogel, Walter, 78532 Tuttlingen (DE); Barth, Sebastian, 78532 Tuttlingen (DE)

(56) Entgegenhaltungen:
- EP-A1- 3 178 434
- EP-A1- 3 514 595
- EP-B1- 1 333 305
- WO-A1-2019/181149
- DE-A1- 10 130 119
- US-A1- 2017 146 780

## Beschreibung

Die vorliegende Erfindung betrifft ein optisches Beobachtungsinstrument, insbesondere ein Operationsmikroskop oder ein Exoskop, nach dem Oberbegriff von Anspruch 1, sowie ein Verfahren zum Erzeugen eines Stereobilds eines Objektfelds.

Zur Beobachtung eines Operationsfelds bei einem chirurgischen Eingriff an einem menschlichen oder tierischen Körper sind optische Beobachtungsinstrumente bekannt, die einem Operateur sowie ggf. weiteren Personen eine genaue oder vergrößerte Betrachtung des Operationsfelds an dem Körper gestatten, wobei zugleich der Zugang zum Operationsfeld nicht wesentlich eingeschränkt wird. Derartige optische Beobachtungsinstrumente können insbesondere als Operationsmikroskop oder als Exoskop ausgebildet sein.

Aus DE 10 2011 054 031 A1 ist eine Vorrichtung zum Beobachten und Beleuchten eines Objektfelds an einem Patienten von einer Stelle abseits des Körpers des Patienten bekannt, die eine Optik zum Beobachten des Objektfelds und eine Beleuchtung zum Beleuchten des Objektfelds aufweist. Die Vorrichtung weist weiterhin einen Schaft auf, an dessen distalem Ende ein gegenüber dem Schaft erweitertes Kopfteil angeordnet ist, in dem eine Beleuchtungseinheit zur Ausleuchtung des Objektfelds angeordnet ist. Der langerstreckte Schaft kann einen Bildweiterleiter aufnehmen, der das Bild des Operationsfelds zu einem proximalen Ende des Schafts weiterleitet. Eine derartige Vorrichtung wird auch als "Exoskop" bezeichnet. Dieses ermöglicht die Beleuchtung und Beobachtung eines Operationsfelds bei einer chirurgischen Operation aus einem Arbeitsabstand von beispielsweise 25 bis 75 cm, so dass der Arbeitsraum des Operateurs durch das Exoskop praktisch nicht eingeschränkt wird. Durch Anschluss einer Videokamera wird es ermöglicht, das Bild des Objektfelds auf einem Bildschirm darzustellen, so dass dieses für den Operateur sowie ggf. weitere Personen ermüdungsfrei beobachtbar ist. Das Exoskop kann durch eine verstellbare Halterung gehalten werden.

Bei der Durchführung einer chirurgischen Operation ist eine räumliche Wahrnehmung des Objektfelds für den operierenden Chirurgen hilfreich. Es ist bekannt, mit einer stereoskopischen Optik, bei der zwei Bilder des Objektfelds aus etwas unterschiedlicher Perspektive aufgenommen werden, eine verbesserte räumliche Wahrnehmung des Obj ektfelds zu ermöglichen. Die beiden Bilder, die gemeinsam das stereoskopische Bild darstellen, werden auch als "Halbbilder" oder als "Stereo-Halbbilder" bezeichnet. Die beiden Halbbilder werden separat dem rechten und dem linken Auge des Operateurs dargestellt, so dass dieser einen räumlichen Eindruck des Objektfelds gewinnen kann. Hierfür kann beispielsweise ein für eine stereoskopische Darstellung geeigneter Monitor vorgesehen sein, etwa ein Bildschirm mit einer wechselnden Polarisierung, wobei der Operateur eine Polarisationsbrille mit unterschiedlichen Polarisierungen der beiden Gläser trägt.

Bei Verwendung einer stereoskopischen Optik stellt sich jedoch das Problem, dass sich bei einer Drehung um eine parallel zur Blickrichtung der Optik stehende Achse nicht nur das mit elektronischen Bildaufnehmern erzeugte Bild und somit auch das auf einem Bildschirm dargestellte Bild des Operationsfelds dreht, sondern zudem auch die Basislinie der stereoskopischen Optik. Das Gleiche gilt, wenn der Operateur seine eigene Position verändert. In beiden Fällen ist für den Operateur die Orientierung im Operationsfeld erschwert oder sogar unmöglich. Eine elektronische Bildaufrichtung ist wiederum nicht möglich, da eine Drehung der beiden Halbbilder des Stereobildes nicht zur Drehung der Stereobasis, also der gedachten Linie zwischen den beiden Mittelpunkten der Aperturen der Stereooptik, führen würde. Der Stereoeindruck ginge daher verloren. Es ist daher notwendig, die Stereobasis der stereoskopischen Optik selbst entsprechend anpassen zu können, um eine Bildaufrichtung oder Ausrichtung des Horizonts zu ermöglichen.

Gemäß DE 10 2013 110 543 A1 umfasst ein Exoskop einen Schaft und eine an einem distalen Ende des Schafts angeordnete Beobachtungsoptik zum Aufnehmen des Bildes des Objektfelds, wobei die Beobachtungsoptik als Stereooptik mit mindestens einem elektronischen Bildaufnehmer zum Aufnehmen eines Stereobilds des Objektfelds ausgebildet ist. Das Exoskop weist eine Optikeinheit auf, die in einem am distalen Ende des Schafts angeordneten Kopfteil angeordnet ist und die die Beobachtungsoptik umfasst. Die Optikeinheit ist um eine zu einer Blickrichtung der Beobachtungsoptik näherungsweise parallele Drehachse drehbar, wobei die Blickrichtung etwa 90° zur Längsachse des Schafts abgewinkelt sein kann. Hierdurch kann das Exoskop bei einer chirurgischen Operation zum Beobachten eines horizontal angeordneten Objektfelds, etwa eines Operationsfelds bei einer chirurgischen Operation am menschlichen Körper, über dem Objektfeld mit senkrecht nach unten gerichteter Blickrichtung positioniert werden. Dabei kann ein stereoskopisches Bild des Objektfelds aufgenommen und bei einem Schwenken des Exoskops sowohl eine Aufrichtung des aufgenommenen und dargestellten Bilds des Objektfelds als auch eine Einstellung der Stereobasis vorgenommen werden.

In EP 1 333 305 B1 wird ein Stereo-Untersuchungssystem zur Abbildung eines Objekts vorgeschlagen, welches eine Objektivanordnung mit einer optischen Achse und einer Objektebene umfasst, wobei die Objektivanordnung ein aus der Objektebene in einen Raumwinkelbereich emittiertes objektseitiges Strahlenbündel empfängt und in ein bildseitiges Strahlenbündel überführt. Weiter umfasst das Stereo-Untersuchungssystem eine Selektoranordnung zur Auswahl von einem ersten und einem zweiten Paar von Teilstrahlenbündeln aus dem bildseitigen Strahlenbündel und eine Bildübertragungsvorrichtung zur Erzeugung von Darstellungen der durch das erste und zweite Paar von Teilstrahlenbündeln bereitgestellten Bilder des Objekts. Die Selektoranordnung ist dazu ausgebildet, einen Strahlquerschnitt wenigstens eines der Teilstrahlenbündel relativ zu einem Strahlquerschnitt des bildseitigen Strahlenbündels zu verlagern, wobei eine Steuerung vorgesehen ist, um die Selektoranordnung zur Verlagerung des Strahlquerschnitts des wenigstens einen Teilstrahlenbündels in Umfangsrichtung um die optische Achse anzusteuern.

Aus der EP3514595A1 ist ein Stereomikroskop bekannt, mit einer Optikeinheit, die relativ zu einem Haltearm um eine Achse senkrecht zur Optikeinheit und um die eigene Längsachse gedreht werden kann.

Die DE10130119A1 offenbart ein Stereomikroskop mit einer Optikeinheit, wobei proximal der Optikeinheit ein Umlenkelement in Form eines Spiegels angeordnet ist.

Zur Beobachtung des aufgenommenen Bilds durch den Operateur hat es sich als vorteilhaft erwiesen, einen oder mehrere Bildschirme (Monitore) gegenüber der Position des Operateurs in erhöhter Stellung anzuordnen, so dass der Operateur über das Operationsfeld, über ggf. verwendete chirurgische Instrumente und über das optische Beobachtungsinstrument hinweg das auf dem Monitor dargestellte Bild betrachten kann. Insbesondere Operationsmikroskope weisen in der Regel jedoch einen länglichen und voluminösen Mikroskopkorpus auf, der eine ungehinderte Sicht über das optische Beobachtungsinstrument verhindert oder eine unvorteilhaft hohe, für den Betrachter unbequeme Position des Monitors erfordern würde. Auch bei Exoskopen kann das Kopfteil das Blickfeld des Operateurs einschränken, wobei die Bauhöhe des Kopfteils insbesondere durch die Länge der Optikeinheit bestimmt wird. Es ist Aufgabe der vorliegenden Erfindung, ein optisches Beobachtungsinstrument, insbesondere ein Operationsmikroskop oder ein Exoskop mit einer Stereooptik anzugeben, das die oben genannten Nachteile nicht aufweist, wobei insbesondere bei einer vertikal nach unten gerichteten Blickrichtung eine vertikale Bauhöhe des optischen Beobachtungsinstruments verringert ist. Ferner ist es Aufgabe der Erfindung, ein verbessertes Verfahren zum Erzeugen eines Stereobilds eines Objektfelds anzugeben.

Diese Aufgabe wird durch ein optisches Beobachtungsinstrument gemäß Anspruch 1 sowie durch ein Verfahren gemäß Anspruch 15 gelöst.

Vorteilhafte Weiterbildungen der Erfindung ergeben sich aus den Unteransprüchen.

Ein erfindungsgemäßes optisches Beobachtungsinstrument ist insbesondere ein medizinisches optisches Beobachtungsinstrument und ist in bevorzugter Weise als Operationsmikroskop oder als Exoskop ausgebildet. Das optische Beobachtungsinstrument kann beispielsweise zum Aufnehmen eines Bildes eines Objektfelds an einem menschlichen oder tierischen Körper von außerhalb des Körpers dienen. Das Objektfeld kann beispielsweise ein Operationsfeld eines chirurgischen Eingriffs sein. Dabei ist das Beobachtungsinstrument vorzugsweise zum Aufnehmen des Bilds des Operationsfelds aus einem Arbeitsabstand ausgebildet, der einem Chirurgen einen ungehinderten Zugang zu dem Operationsfeld bei dem chirurgischen Eingriff erlaubt. Der Arbeitsabstand kann beispielsweise im Bereich von etwa 10 bis 75 cm, vorzugsweise etwa 15 bis 50 cm, liegen. Wenn im Folgenden von einem "Benutzer" des optischen Beobachtungsinstruments die Rede ist, so ist hiermit insbesondere ein operierender Chirurg oder Operateur gemeint; ein Benutzer kann jedoch auch beispielsweise eine Person sein, die den Operateur bei dem Eingriff unterstützt oder ein weiterer Beobachter des Eingriffs, oder auch ein Benutzer bei nicht-medizinischen Anwendungen des optischen Beobachtungsinstruments.

Das erfindungsgemäße optische Beobachtungsinstrument umfasst eine Optikeinheit, die eine Objektivanordnung und mindestens einen elektronischen Bildaufnehmer aufweist. Die Optikeinheit ist zum Aufnehmen eines Stereobilds eines Objektfelds, beispielsweise eines Objektfelds an einem menschlichen oder tierischen Körper, mit dem mindestens einen elektronischen Bildaufnehmer und somit als Stereooptik ausgebildet. Die Optikeinheit weist hierfür einen ersten Stereokanal mit einem ersten Strahlengang und einen zweiten Stereokanal mit einem zweiten Strahlengang auf zum Aufnehmen eines ersten und eines zweiten Stereo-Halbbilds des Objektfelds. Dabei kann es vorgesehen sein, dass das Objektfeld mittels der Objektivanordnung über den ersten Strahlengang auf einen ersten elektronischen Bildaufnehmer abgebildet wird, um das erste Stereo-Halbbild aufzunehmen, und das Objektfeld mittels der Objektivanordnung über den zweiten Strahlengang auf einen zweiten elektronischen Bildaufnehmer abgebildet wird, um das zweite Stereo-Halbbild des Objektfelds aufzunehmen. Anstelle eines ersten und eines zweiten elektronischen Bildaufnehmers kann auch beispielsweise ein einziger Bildaufnehmer zur Aufnahme des ersten und des zweiten Stereo-Halbbilds dienen, wobei das erste und das zweite Stereo-Halbbild des Objektfelds auf unterschiedlichen Bereichen des Bildaufnehmers abgebildet werden. Der mindestens eine elektronische Bildaufnehmer ist insbesondere ein CCD- oder ein MOSFET-Bildsensor.

Der erste und der zweite Strahlengang verlaufen durch die Objektivanordnung. Die Objektivanordnung dient insbesondere zum Abbilden des Objektfelds auf dem ersten und dem zweiten Bildaufnehmer oder entsprechenden Bereichen eines einzigen Bildaufnehmers über den ersten bzw. den zweiten Strahlengang. Die Objektivanordnung kann gemeinsame abbildende optische Elemente umfassen, die dem ersten und dem zweiten Stereokanal gemeinsam sind, sowie ggf. weitere abbildende optische Elemente, die jeweils nur dem ersten bzw. dem zweiten Stereokanal zugeordnet sind, wie etwa eine oder mehrere Frontlinsen oder eine oder mehrere Zoomoptiken. Die Objektivanordnung kann aber auch ein Objektiv des ersten und ein Objektiv des zweiten Stereokanals umfassen, welche separat voneinander ausgebildet sein können. Die Objektivanordnung kann als eine Baugruppe ausgebildet sein und somit eine Objektiveinheit der Optikeinheit bilden. Die Objektivanordnung ist insbesondere als Linsensystem ausgebildet, kann aber auch reflektierende optische Elemente umfassen. Weiter kann die Objektivanordnung ein oder mehrere Filter umfassen, die dem ersten und dem zweiten Stereokanal gemeinsam oder jeweils nur dem ersten bzw. dem zweiten Stereokanal zugeordnet sind, beispielsweise ein oder mehrere Fluoreszenzfilter. Die Filter können insbesondere zur Beobachtung und Aufnahme von Fluoreszenzlicht und zur Filterung des Anregungslichts der Fluoreszenz eingerichtet sein. Das Beobachtungsinstrument kann damit zur insbesondere gleichzeitigen Beobachtung von Fluoreszenz durch die Farbstoffe ICG (Indocyaningrün), Fluorescein und PPIX (Protoporphyrin-IX) ausgebildet sein.

Die Objektivanordnung weist eine Achse auf, die beispielsweise eine optische Achse einer Objektivlinse oder eines Linsensystems sein kann, die bzw. das von dem ersten und dem zweiten Strahlengang durchsetzt wird. In dem Fall, dass der erste und der zweite Stereokanal jeweils ein separates Objektiv aufweisen, ist die Achse insbesondere eine Mittelachse der beiden Strahlengänge bzw. der optischen Achsen der beiden Objektive. Der erste und der zweite Strahlengang sind quer zur Achse der Objektivanordnung gegeneinander versetzt, wobei durch die Verbindungslinie zwischen dem ersten und dem zweiten Strahlengang, insbesondere in einem objektseitigen Element der Objektivanordnung, eine Stereobasis der Stereooptik bestimmt wird. Objektseitig der Objektivanordnung können der erste und der zweite Strahlengang winklig zueinander verlaufen unter Einschluss eines Stereowinkels, so dass sich die Strahlengänge bei einem bevorzugten Arbeitsabstand, beispielsweise in einem Abstand von ca. 25 cm, treffen oder zumindest überlappen. Der erste und der zweite Strahlengang können objektseitig der Objektivanordnung aber auch parallel zueinander verlaufen; der Arbeitsabstand und somit der Stereowinkel ergibt sich dann insbesondere durch den eingestellten Fokus und/oder durch eine Disparität der aufgenommenen Stereo-Halbbilder bzw. umgekehrt. Die Achse der Objektivanordnung kann insbesondere eine Winkelhalbierende zwischen den objektseitigen Abschnitten des ersten und des zweiten Strahlengangs sein.

Weiter umfasst das optische Beobachtungsinstrument eine Haltevorrichtung, die vorzugsweise verstellbar ausgebildet ist, wobei die Optikeinheit an der Haltevorrichtung um eine erste Drehachse drehbar gelagert ist. Die erste Drehachse stimmt zumindest näherungsweise mit der Achse der Objektivanordnung überein, die beispielsweise die optische Achse einer gemeinsamen Objektivlinse und/oder eine Mittelachse zwischen den optischen Achsen des ersten und des zweiten Stereokanals sein kann. Die Haltevorrichtung kann derart ausgebildet sein, dass sie an einem Operationstisch befestigt werden kann. Hierdurch kann erreicht werden, dass die Optikeinheit relativ zum Operationstisch um die optische Achse drehbar ist. Durch die drehbare Lagerung kann eine Rotation der Stereobasis ermöglicht werden, so dass die Stereobasis entsprechend dem Standort eines Benutzers und der Ausrichtung des optischen Beobachtungsinstruments eingestellt werden kann, um dem Benutzer einen natürlichen Stereoeindruck bei aufgerichtetem oder zum Horizont ausgerichteten Bild und eine einfache Orientierung innerhalb des Objektfelds zu ermöglichen. Eine Drehung der Optikeinheit entspricht also vorzugsweise einer Drehung der Stereobasis um eine Achse senkrecht zur Stereobasis.

Erfindungsgemäß hat das optische Beobachtungsinstrument eine gegenüber der Achse der Objektivanordnung abgewinkelte Blickrichtung und umfasst ein Umlenkelement, das objektseitig der Objektivanordnung angeordnet ist und das zur Umlenkung des ersten und des zweiten Strahlengangs in die Objektivanordnung ausgebildet und angeordnet ist. Das optische Umlenkelement kann eine oder mehrere reflektierende Flächen umfassen. Die Blickrichtung ist dabei insbesondere eine mittlere Richtung zwischen den Blickrichtungen des ersten und des zweiten Stereokanals bzw. der objektseitigen Abschnitte des ersten und des zweiten Strahlengangs. Das optische Umlenkelement ist somit zur Umlenkung des ersten und des zweiten Strahlengangs aus ihren jeweiligen Blickrichtungen in die Objektivanordnung und damit in die optischen Elemente des ersten und des zweiten Stereokanals zur Erzeugung des ersten und des zweiten Stereo-Halbbilds angeordnet und ausgebildet, wobei die mittlere Richtung zwischen den Blickrichtungen des ersten und des zweiten Strahlengangs der Blickrichtung des Beobachtungsinstruments entspricht, die gegenüber der Achse der Obj ektivanordnung abgewinkelt ist. Entsprechend kann als Blickachse des optischen Beobachtungsinstruments, deren Richtung der Blickrichtung entspricht, insbesondere eine mittlere Achse oder eine Winkelhalbierende zwischen den objektseitigen Abschnitten des ersten und des zweiten Strahlengangs angesehen werden, die sich bei dem bevorzugten Arbeitsabstand treffen. Die objektseitigen Abschnitte des ersten und des zweiten Strahlengangs bzw. deren jeweilige Blickrichtungen bilden dann mit der Blickachse jeweils einen Winkel, der dem halben Stereowinkel entspricht. In dem Fall, dass die objektseitigen Abschnitte des ersten und des zweiten Strahlengangs parallel zueinander verlaufen, entspricht die Blickrichtung des optischen Beobachtungsinstruments den Blickrichtungen des ersten und des zweiten Strahlengangs, und die Blickachse ist insbesondere eine mittlere Achse zwischen den objektseitigen Abschnitten des ersten und des zweiten Strahlengangs, welche gegenüber der Achse der Objektivanordnung abgewinkelt ist.

Dies bedeutet insbesondere, dass durch das optische Umlenkelement ein objektseitiger Strahlengang des optischen Beobachtungsinstruments aus einer gegenüber der Achse der Objektivanordnung abgewinkelten Blickrichtung zumindest näherungsweise in Richtung der Achse der Objektivanordnung in die Objektivanordnung umgelenkt wird. Der objektseitige Strahlengang kann durch vom Objektfeld ausgehende, in einen kegelförmigen Raumwinkelbereich, der die Blickachse umgibt, emittierte Strahlen gebildet werden, welche durch das Umlenkelement zur Objektivanordnung umgelenkt werden, wobei diejenigen dieser Strahlen, die den ersten und den zweiten Strahlengang bilden und in die optischen Elemente des ersten bzw. des zweiten Stereokanals eintreten, zur Erzeugung des ersten bzw. des zweiten Stereo-Halbbilds auf dem mindestens einen elektronischen Bildaufnehmer abgebildet werden. Die Blickachse ist dabei die Achse des kegelförmigen Raumwinkelbereichs, der einen etwa dem Stereowinkel entsprechenden Öffnungswinkel hat. Insbesondere fallen die vom Umlenkelement umgelenkten, aus dem Objektfeld kommenden Strahlen in einem entsprechenden Raumwinkelbereich, der die Achse der Obj ektivanordnung umgibt, auf die Objektivanordnung ein, so dass jeweilige Anteile des einfallenden Lichts in den ersten und den zweiten Stereokanal gelangen und auf den betreffenden Bildaufnehmern bzw. Bereichen des Bildaufnehmers abgebildet werden.

Dadurch, dass das optische Beobachtungsinstrument, das insbesondere ein Stereo-Operationsmikroskop oder ein Stereo-Exoskop ist, eine Blickrichtung hat, die zur Achse der Objektivanordnung abgewinkelt ist, kann eine besonders günstige Handhabung ermöglicht werden. Insbesondere kann hierdurch erreicht werden, dass eine Baulänge der Optikeinheit in Richtung der Achse der Objektivanordnung sich nicht in der Blickrichtung erstreckt, wobei die Baulänge der Optikeinheit unter anderem durch eine Länge des ersten und des zweiten Strahlengangs bestimmt sein kann und somit durch die an das optische Beobachtungsinstrument gestellten optischen Anforderungen vorgegeben sein kann. Somit ist etwa dann, wenn die Blickrichtung des optischen Beobachtungsinstruments vertikal nach unten auf ein Operationsfeld an einem menschlichen oder tierischen Körper gerichtet ist, die vertikale Bauhöhe des optischen Beobachtungsinstruments, beispielsweise die Bauhöhe eines Kopfteils eines Stereo-Exoskops, nicht durch die Baulänge der Optikeinheit vorgegeben, sondern kann geringer gewählt werden. Hierdurch kann eine Einschränkung des Blickfelds eines Operateurs beim Blick auf einen gegenüberliegend angeordneten Monitor reduziert oder vermieden werden.

Ferner kann dadurch, dass distalseitig des Objektivs ein optisches Umlenkelement angeordnet ist, auf einfache Weise eine relativ zur Achse der Objektivanordnung abgewinkelte Blickrichtung des optischen Beobachtungsinstruments realisiert werden. Dadurch, dass die Optikeinheit und damit die durch die Anordnung des ersten und des zweiten Stereokanals der Optikeinheit definierte Stereobasis zumindest näherungsweise um die Achse der Objektivanordnung bzw. um die Mittelachse der Strahlengänge gedreht werden kann, kann dabei auf besonders einfache und vorteilhafte Weise eine Anpassung der Richtung der Stereobasis an einen Standort des Benutzers und die Ausrichtung des Beobachtungsinstruments sowie eine Aufrichtung des Stereobilds ermöglicht werden. So kann beispielsweise in dem Fall, dass die Blickrichtung des optischen Beobachtungsinstruments auf ein Operationsfeld bzw. einen Körperbereich eines Patienten gerichtet ist, der auf einem Operationstisch liegt, für unterschiedliche Standorte des Benutzers die Stereobasis auf einfache Weise derart eingestellt werden, dass das Stereobild einen natürlichen räumlichen und aufgerichteten Eindruck des Operationsfelds bzw. des beobachteten Bereichs der Körperoberfläche ergibt. Somit kann es auf diese Weise ermöglicht werden, dass der Operateur die Optikeinheit frei positionieren und sodann die Stereobasis entsprechend seinem Standort und dem natürlichen oder bevorzugten Horizont einstellen kann, wobei zugleich eine wesentliche Einschränkung seines Blickfelds vermieden werden kann.

Vorliegend ist die Optikeinheit relativ zur Haltevorrichtung und relativ zum Umlenkelement drehbar gelagert.

Vorzugsweise ist die Blickrichtung zumindest näherungsweise um 90° gegenüber der Achse der Objektivanordnung abgewinkelt. Hierdurch kann es ermöglicht werden, dass bei einer Blickrichtung, die näherungsweise vertikal nach unten gerichtet ist, was für einen chirurgischen Eingriff in der Regel eine besonders günstige Blickrichtung ist, die Optikeinheit sich in etwa horizontaler Richtung erstreckt und um eine näherungsweise horizontale Achse drehbar ist, um die Richtung der Stereobasis einzustellen. Dadurch kann erreicht werden, dass einerseits das Objektfeld frei zugänglich ist, insbesondere ein Operationsfeld für den Operateur frei zugänglich ist, und andererseits ein weitestgehend unbehinderter Blick über das optische Beobachtungsinstrument hinweg auf einen Monitor möglich ist, der beispielsweise dem Operateur gegenüber neben dem Operationstisch angeordnet sein kann.

In besonders bevorzugter Weise weist die Optikeinheit ein länglich ausgebildetes oder langerstrecktes Gehäuse auf, das im Folgenden als Optikgehäuse bezeichnet wird, und welches sich zumindest näherungsweise in Richtung der Achse der Obj ektivanordnung erstreckt. Dabei sind insbesondere optische Elemente der Optikeinheit, insbesondere die Objektivanordnung, bevorzugt alle optischen Elemente oder alle Linsen der Optikeinheit, in dem Optikgehäuse aufgenommen. Eine längliche oder langerstreckte Ausbildung der Optikeinheit ermöglicht eine besonders vorteilhafte Ausgestaltung des ersten und des zweiten Strahlengangs zur Erzeugung der beiden Stereo-Halbbilder. Gemäß dieser Ausführungsform ist somit eine Längsrichtung des Optikgehäuses zumindest näherungsweise parallel zur Achse der Objektivanordnung, und die erste Drehachse, bezüglich derer die Optikeinheit drehbar an der Haltevorrichtung gelagert ist, ist zumindest näherungsweise parallel zur Längsrichtung des Optikgehäuses. Das optische Beobachtungsinstrument kann daher beispielsweise derart über einem Operationstisch gehalten werden, dass die Blickrichtung näherungsweise in vertikaler Richtung nach unten gerichtet ist und eine Längsrichtung der Optikeinheit bzw. des Optikgehäuses, die näherungsweise parallel zur Achse der Objektivanordnung und der ersten Drehachse ist, horizontal gerichtet ist. Hierdurch wird einerseits eine besonders vorteilhafte Bauform der Optikeinheit geschaffen und andererseits einem Benutzer ermöglicht, ohne wesentliche Einschränkung des Arbeitsbereichs und des Gesichtsfelds über das optische Beobachtungsinstrument hinweg auf einen Monitor zu blicken. Das Optikgehäuse kann beispielsweise einen Mikroskopkorpus eines Operationsmikroskops darstellen.

Gemäß einer besonders bevorzugten Ausführungsform der Erfindung ist das Optikgehäuse der Optikeinheit um die erste Drehachse relativ zur Haltevorrichtung drehbar gelagert und enthält optische Elemente der Optikeinheit, insbesondere die Objektivanordnung, bevorzugt alle optischen Elemente oder alle Linsen der Optikeinheit, und den mindestens einen elektronischen Bildaufnehmer, beispielsweise mindestens zwei elektronische Bildaufnehmer, zur Aufnahme des ersten und des zweiten Stereo-Halbbilds, wobei ferner die Blickrichtung des optischen Beobachtungsinstruments gegenüber der ersten Drehachse wie zuvor beschrieben um etwa 90° abgewinkelt ist.

Gemäß einer bevorzugten Ausführungsform der Erfindung umfasst die Objektivanordnung eine objektseitige Frontlinse, wobei der erste und der zweite Strahlengang durch die Frontlinse verlaufen. Die Achse der Objektivanordnung ist in diesem Fall insbesondere eine optische Achse oder eine Mittelachse der Frontlinse, welche vorzugsweise zugleich eine Mittelachse zwischen dem ersten und dem zweiten Strahlengang bzw. zwischen optischen Achsen des ersten und des zweiten Strahlengangs sein kann. Der erste und der zweite Strahlengang bzw. deren optische Achsen verlaufen insbesondere symmetrisch zur Achse der Objektivanordnung durch die Frontlinse, wobei die objektseitigen Abschnitte des ersten und des zweiten Strahlengangs parallel oder winklig zueinander verlaufen können und näherungsweise den Stereowinkel einschließen und die bildseitig der Frontlinse anschließenden Abschnitte des ersten und des zweiten Strahlengangs parallel zueinander und zur Achse der Obj ektivanordnung verlaufen können. Gemäß dieser Ausführungsform der Erfindung umfasst das optische Beobachtungsinstrument somit eine Optikeinheit mit einer Objektivanordnung, die eine objektseitige Frontlinse aufweist, und mindestens einem elektronischen Bildaufnehmer, wobei die Optikeinheit einen ersten Stereokanal mit einem ersten Strahlengang und einen zweiten Stereokanal mit einem zweiten Strahlengang zum Aufnehmen eines Stereobilds eines Objektfelds mit dem mindestens einen elektronischen Bildaufnehmer aufweist und wobei der erste und der zweite Strahlengang durch die Frontlinse verlaufen, und eine Haltevorrichtung, an der die Optikeinheit um eine erste Drehachse drehbar gelagert ist, die zumindest näherungsweise mit der optischen Achse der Frontlinse übereinstimmt, wobei das optische Beobachtungsinstrument ein objektseitig der Frontlinse angeordnetes Umlenkelement zur Umlenkung des ersten und des zweiten Strahlengangs aus ihren jeweiligen Blickrichtungen in die Objektivanordnung umfasst, wobei eine mittlere Richtung der Blickrichtungen, die die Blickrichtung des Beobachtungsinstruments ist, gegenüber der optischen Achse der Frontlinse abgewinkelt ist. Hierdurch kann eine besonders einfache Ausgestaltung mit einem großen Drehwinkelbereich der Optikeinheit ermöglicht werden.

In besonders bevorzugter Weise umfasst das optische Umlenkelement einen, vorzugsweise genau einen, schräg zur Achse der Objektivanordnung stehenden Planspiegel. Insbesondere kann das Umlenkelement als ein solcher Planspiegel ausgebildet sein. In dem Fall, dass die Blickrichtung des optischen Beobachtungsinstruments näherungsweise um 90° gegenüber der Achse der Objektivanordnung abgewinkelt ist, steht die Flächennormale des Planspiegels näherungsweise in einem Winkel von 45° zur Achse der Objektivanordnung. Der Planspiegel weist vorzugsweise nur eine einzige reflektierende Fläche auf, um die Entstehung doppelter Reflexionen zu vermeiden. Dadurch, dass das Umlenkelement als schräg zur Achse der Objektivanordnung, beispielsweise schräg zur optischen Achse der Frontlinse, angeordneter Spiegel ausgebildet ist, kann eine besonders leichte und einfache Bauform ermöglicht werden. Alternativ könnte das Umlenkelement als Prisma oder Prismenanordnung ausgebildet sein.

Vorzugsweise ist das optische Umlenkelement an der Haltevorrichtung angeordnet. Das optische Umlenkelement kann beispielsweise feststehend, insbesondere drehfest an der Haltevorrichtung angeordnet sein. Durch Drehung der Optikeinheit um die erste Drehachse wird die Optikeinheit, insbesondere das Optikgehäuse, somit zugleich entsprechend relativ zum optischen Umlenkelement gedreht. Dies ermöglicht eine besonders einfache und stabile Bauform und eine weiter vereinfachte Bedienung des Beobachtungsinstruments. Außerdem wird so die Ausrichtung der Stereobasis an den gewünschten Horizont durch den Anwender ermöglicht, ohne die durch das Umlenkelement bestimmte Blickrichtung des Instruments zu verändern.

Alternativ kann das optische Umlenkelement relativ zur Haltevorrichtung und zur Optikeinheit, insbesondere relativ zum Optikgehäuse, drehbar angeordnet sein, insbesondere um die erste Drehachse drehbar. Das optische Umlenkelement kann beispielsweise entsprechend drehbar an der Haltevorrichtung angeordnet sein. Hierdurch kann eine zusätzliche Einstellmöglichkeit zur Verstellung der Blickrichtung des Beobachtungsinstruments und zur Wahl des Objektfelds geschaffen werden.

In vorteilhafter Weise kann das optische Umlenkelement derart an der Haltevorrichtung angeordnet sein, dass es von einem Benutzer bei der Bedienung oder beim Konfigurieren des optischen Beobachtungsinstruments ganz oder teilweise abgenommen werden kann. Durch eine derartige lösbare Befestigung des Umlenkelements an der Haltevorrichtung kann die Handhabung und die Reinigung des Beobachtungsinstruments erleichtert werden sowie eine Benutzung des Beobachtungsinstruments auch ohne das Umlenkelement ermöglicht werden.

Gemäß einer vorteilhaften Ausführungsform der Erfindung kann es vorgesehen sein, dass das optische Umlenkelement ein erstes und ein zweites Spiegelelement umfasst, wobei das zweite Spiegelelement objektseitig des ersten Spiegelelements angeordnet ist, so dass der erste und der zweite Strahlengang über das erste und das zweite Spiegelelement verlaufen und durch diese jeweils eine Umlenkung erfahren. Durch die aufeinander folgenden Umlenkungen durch das zweite und danach durch das erste Spiegelelement werden der erste und der zweite Strahlengang aus ihren jeweiligen Blickrichtungen in die Objektivanordnung und somit in den ersten und den zweiten Stereokanal umgelenkt. Das zweite Spiegelelement ist schwenkbar um eine zumindest näherungsweise zur ersten Drehachse senkrechte Schwenkachse gelagert. Insbesondere kann ein Spiegelgehäuse des zweiten Spiegelelements drehbar an einem Spiegelgehäuse des ersten Spiegelelements gelagert sein. Das erste und das zweite Spiegelelement können jeweils als schräg stehende Planspiegel ausgebildet sein und jeweils zur Umlenkung des Strahlengangs um etwa 90° angeordnet sein; so kann der Planspiegel, der das erste Spiegelelement bildet, beispielsweise um 45° schräg zur Achse der Objektivanordnung stehen und einen aus Richtung der zweiten Drehachse einfallenden Strahl in die Achse der Objektivanordnung umlenken, und der Planspiegel, der das zweite Spiegelelement bildet, etwa um 45° schräg zur zweiten Drehachse. Auf diese Weise kann eine weitere Einstellmöglichkeit zur Verstellung der Blickrichtung des optischen Beobachtungsinstruments geschaffen werden, so dass das beobachtete Objektfeld verändert werden kann, ohne dass in jedem Fall die räumliche Anordnung der Optikeinheit geändert werden muss. In dem Fall, dass das erste Spiegelelement um die erste Drehachse drehbar an der Haltevorrichtung gelagert ist, kann eine Verstellung der Blickrichtung des optischen Beobachtungsinstruments um zwei Achsen ermöglicht und eine zusätzliche Einstellmöglichkeit zur Wahl des Objektfelds geschaffen werden kann.

Gemäß einer besonders bevorzugten Ausführungsform der Erfindung ist die Optikeinheit mit einem um eine zweite Drehachse drehbaren Bedienelement drehgekoppelt, wobei die zweite Drehachse zumindest näherungsweise parallel zur Blickrichtung des optischen Beobachtungselements gerichtet ist. Insbesondere ist das drehbare Bedienelement derart angeordnet, dass die zweite Drehachse etwa mit der Blickachse oder der Winkelhalbierenden zwischen den objektseitigen Abschnitten des ersten und des zweiten Strahlengangs übereinstimmt. Dabei ist eine Abweichung zwischen der zweiten Drehachse und der Blickrichtung bzw. der Blickachse vorzugsweise möglichst gering, kann aber auch beispielsweise bis zu ca. 20° oder maximal bis zu ca. 45° betragen. Das Bedienelement kann beispielsweise als Drehrad oder als Drehknopf ausgebildet sein, das bzw. der manuell drehbar ist, so dass durch Drehung des Bedienelements um die zweite Drehachse die Optikeinheit manuell um die erste Drehachse gedreht werden kann. Gemäß diesem Aspekt der Erfindung ist es erkannt worden, dass dadurch, dass die Drehachse des Bedienelements zumindest näherungsweise parallel zur Blickrichtung oder mit der Blickachse übereinstimmend ist, eine einfache und intuitive Bedienung des optischen Beobachtungsinstruments zum Einstellen der Ausrichtung der Stereobasis ermöglicht wird.

In besonders vorteilhafter Weise kann das Bedienelement auf einer der Richtung zum Objektfeld entgegengesetzten Seite an dem optischen Umlenkelement, beispielsweise an einem Spiegelgehäuse des Umlenkelements, angeordnet sein. Hierdurch wird einerseits eine einfache und sichere mechanische Befestigung des drehbaren Bedienelements ermöglicht und andererseits eine besonders einfache und intuitive Bedienung ermöglicht.

Vorzugsweise ist das drehbare Bedienelement über ein Getriebe mit der Optikeinheit drehgekoppelt. Das Getriebe kann insbesondere ein erstes und ein zweites Zahnrad umfassen oder im Wesentlichen aus dem ersten und dem zweiten Zahnrad bestehen, wobei das erste Zahnrad drehfest mit dem drehbaren Bedienelement gekoppelt ist und das zweite Zahnrad, das mit dem ersten Zahnrad kämmt, mit der Optikeinheit drehfest gekoppelt ist. In vorteilhafter Weise können das erste und das zweite Zahnrad jeweils als Kegelzahnrad ausgebildet sein. Das erste Zahnrad kann somit mittels des Bedienelements um die zweite Drehachse gedreht werden, und das zweite Zahnrad gemeinsam mit der Optikeinheit um die erste Drehachse gedreht werden, so dass durch Drehen des Bedienelements die Optikeinheit zur Einstellung der Stereobasis und zum Aufrichten der Stereo-Halbbilder gedreht werden kann. In besonders bevorzugter Weise können das erste und das zweite Zahnrad eine gleiche Anzahl von Zähnen aufweisen, wobei weiter vorzugsweise der Durchmesser des ersten und des zweiten Zahnrads näherungsweise gleich ist. Auf diese Weise wird es ermöglicht, durch eine Drehung des drehbaren Bedienelements eine gleich große Drehung der Optikeinheit auszuführen. Hierdurch kann die Bedienung weiter vereinfacht und intuitiver gestaltet werden. Insbesondere kann die Bedienung zur Ausrichtung der Stereobasis und zum Aufrichten des Stereobilds in einer Weise erfolgen, die derjenigen bei einem gattungsgemäßen optischen Beobachtungsinstrument entspricht.

Alternativ oder zusätzlich kann es vorgesehen sein, dass die Optikeinheit von einem Benutzer des optischen Beobachtungsinstruments ergriffen und manuell um die erste Drehachse gedreht werden kann, beispielsweise das Optikgehäuse direkt ergriffen und gedreht werden kann. Ein wie zuvor beschrieben ausgestaltetes Bedienelement ist in diesem Fall nicht unbedingt notwendig. Hierdurch können ein einfacher Aufbau sowie ebenfalls eine einfache Bedienung des optischen Beobachtungsinstruments zum Einstellen der Ausrichtung der Stereobasis ermöglicht werden.

In vorteilhafter Weise kann objektseitig des Umlenkelements ein Deckglas angeordnet sein. Hierdurch kann das Umlenkelement vor Beschädigungen und Verunreinigungen geschützt werden, beispielsweise gegen Staubablagerungen oder Flüssigkeitsspritzer. In weiter vorteilhafter Weise kann das Deckglas um die zweite Drehachse drehbar und mit dem drehbaren Bedienelement drehgekoppelt sein, beispielsweise über eine parallel zur zweiten Drehachse verlaufende Verbindungswelle, die vom zweiten Bedienelement angetrieben wird und eine entsprechende Drehung des Deckglases bewirkt. Hierdurch kann es beispielsweise ermöglicht werden, zusätzliche optische Elemente objektseitig am oder vor dem Deckglas anzubringen und bei einer Änderung der Stereobasis mitzudrehen. Das Deckglas kann schräg zur Blickachse des optischen Beobachtungsinstruments angeordnet sein, insbesondere um störendes Streulicht zu vermeiden.

Vorzugsweise umfasst die Optikeinheit eine Beleuchtungsoptik zur Beleuchtung des Objektfelds mit einem Beleuchtungsstrahlengang, der durch die Objektivanordnung hindurch verläuft. Die Beleuchtungsoptik kann eine Lichtquelle, wie etwa eine lichtemittierende Diode (LED), und eine Kollimatoroptik umfassen. Die Beleuchtungsoptik kann andererseits auch etwa als Lichtanschluss zum Anschließen einer externen Lichtquelle mittels eines Lichtleitkabels mit einer entsprechenden Kollimatoroptik ausgebildet sein. Die Objektivanordnung kann einen Strahlteiler aufweisen zum Einkoppeln der Beleuchtungsstrahlung in den Strahlengang der Optikeinheit näherungsweise in Richtung der Achse der Objektivanordnung zur Beleuchtung des Objektfelds, oder die Beleuchtungsstrahlung kann beispielsweise ohne Strahlteiler seitlich näherungsweise in Richtung auf das Objektfeld in die Objektivanordnung eingestrahlt werden. Dadurch, dass die von der Beleuchtungsoptik abgegebene Beleuchtungsstrahlung durch die Objektivanordnung hindurch in Richtung auf das Objektfeld projiziert wird und somit ebenfalls über das Umlenkelement umgelenkt wird, kann es auf besonders einfache Weise erreicht werden, dass bei jeder möglichen Ausrichtung der Optikeinheit und, sofern das optische Umlenkelement zur Veränderung der Blickrichtung drehbar ist, des Umlenkelements die Beleuchtungsstrahlung stets auf das Objektfeld gerichtet ist.

Alternativ kann die Beleuchtungsoptik am Umlenkelement angeordnet sein und ein Beleuchtungsstrahlengang direkt oder über einen Strahlteiler auf das Objektfeld gerichtet werden. Auch hierdurch kann eine Ausrichtung der Beleuchtungsstrahlung auf das Objektfeld ermöglicht werden.

Vorzugsweise umfasst die Haltevorrichtung einen Haltebügel, der die Optikeinheit übergreift, wobei das Umlenkelement an einem objektseitigen Ende des Haltebügels angeordnet ist. Insbesondere kann der Haltebügel ein Drehlager aufweisen, an dem die Optikeinheit um die erste Drehachse drehbar gelagert ist, wobei das Drehlager am objektseitigen Ende des Haltebügels bzw. der Optikeinheit angeordnet ist und von der Achse der Objektivanordnung sowie von dem ersten und dem zweiten Strahlengang durchsetzt wird. Der Haltebügel übergreift die Optikeinheit vorzugsweise in Längsrichtung oder entlang der oder parallel zur optischen Achse der Optikeinheit. Der Haltebügel kann ein weiteres Drehlager aufweisen, das am objektfernen Ende des Haltebügels bzw. der Optikeinheit angeordnet ist, und durch das beispielsweise Verbindungskabel geführt sein können, wobei die Optikeinheit zwischen den beiden Drehlagern um die erste Drehachse drehbar gelagert ist. Das bzw. die Drehlager kann bzw. können beispielsweise als Gleitlager ausgebildet sein. Das Umlenkelement ist insbesondere obj ektseitig des objektseitigen Drehlagers angeordnet und drehbar oder fest mit dem Haltebügel verbunden. Alternativ kann die Haltevorrichtung beispielsweise als Rohr oder als Schaft ausgebildet sein, innerhalb dessen die Optikeinheit aufgenommen und drehbar gelagert ist, wobei die erste Drehachse der Längsachse des Rohrs bzw. des Schafts entspricht. Weiter alternativ kann die Haltevorrichtung eine Stange aufweisen, die zumindest abschnittsweise durch die Optikeinheit verläuft und auf der die Optikeinheit drehbar gelagert ist, wobei die erste Drehachse der Längsachse der Stange entspricht. Hierdurch kann eine kompakte und robuste Halterung der Optikeinheit geschaffen werden.

In vorteilhafter Weise kann an dem am objektseitigen Ende des Haltebügels bzw. der Optikeinheit angeordneten Drehlager und/oder an dem am objektfernen Ende angeordneten Drehlager eine Arretierungseinrichtung vorgesehen sein, durch die die Optikeinheit bzw. das Optikgehäuse in einer jeweiligen eingestellten Drehposition gehalten wird. Die Arretierungseinrichtung kann beispielsweise durch einen Reibschluss des Drehlagers oder durch eine Ratsche bzw. eine Sperrklinke gebildet werden. Dabei kann die Sperrklinke insbesondere derart federbelastet sein, dass die Optikeinheit durch die Federkraft in der eingestellten Drehposition gehalten wird, jedoch durch eine manuelle Drehung der Optikeinheit die Sperrklinke aus einer entsprechenden Raste gegen die Federkraft ausgehoben wird.

In weiter vorteilhafter Weise kann die Haltevorrichtung einen Haltewinkel umfassen, wobei der Haltebügel drehbar an dem Haltewinkel angeordnet ist. Insbesondere ist der Haltebügel derart drehbar an dem Haltewinkel der Haltevorrichtung gelagert, dass die Optikeinheit um eine dritte Achse drehbar ist, die näherungsweise senkrecht zur ersten Drehachse steht und zudem näherungsweise durch einen Schwerpunkt der Optikeinheit, des Haltebügels und weiterer am Haltebügel angeordneter Bauelemente, etwa des optischen Umlenkelements, verläuft. Weiter vorzugsweise umfasst die Haltevorrichtung einen Haltearm, wobei der Haltewinkel drehbar und/oder längsverschiebbar an dem Haltearm angeordnet ist. Der Haltewinkel ist insbesondere derart ausgebildet und drehbar an dem Haltearm angeordnet, dass eine Drehachse der Lagerung des Haltewinkels an dem Haltearm senkrecht zur dritten Achse steht und diese näherungsweise im Schwerpunkt der Optikeinheit, des Haltebügels und der weiteren am Haltebügel angeordneten Bauelemente schneidet. Hierdurch kann eine besonders einfache Verstellung der Position und der Orientierung der Optikeinheit im Raum ermöglicht werden, wobei bereits eine geringe Haltekraft ausreicht, um die Optikeinheit in einer eingestellten Orientierung zu halten. Die Optikeinheit kann somit vom Benutzer leicht in eine solche Lage gebracht und durch die Haltevorrichtung in dieser gehalten werden, dass einerseits eine ungehinderte Beobachtung des Operationsfelds ermöglicht wird und andererseits das Blickfeld des Benutzers beim Blick auf einen Monitor möglichst wenig eingeschränkt ist.

Alternativ kann der Haltebügel am Haltearm angeordnet sein, vorzugsweise drehbar am Haltearm angeordnet. Insbesondere kann der Haltebügel mit seinem obj ektfemen Ende am Haltearm angeordnet sein, wobei der Haltebügel direkt oder über ein Drehgelenk mit dem Haltearm verbunden ist. Ein wie zuvor beschrieben ausgestalteter Haltewinkel ist in diesem Fall nicht notwendig.

Die Haltevorrichtung kann in vorteilhafter Weise derart ausgebildet sein, dass eine eingestellte Position und/oder Orientierung der Optikeinheit durch Reibschluss, Kraftschluss, Formschluss, elektromagnetisch und/oder motorisch fixierbar ist. Insbesondere kann es vorgesehen sein, dass eine Orientierung der Optikeinheit relativ zum Haltebügel, eine Drehposition des Haltebügels relativ zum Haltewinkel und/oder eine Drehposition und/oder Verschiebestellung des Haltewinkels relativ zum Haltearm manuell vorgenommen werden können und durch einen Reibschluss oder auch mittels einer elektromagnetischen Bremseinrichtung fixiert gehalten werden können. Die Haltevorrichtung kann aber auch in entsprechender Weise motorisch verstellbar ausgebildet sein, und die entsprechende Position bzw. Orientierung der Optikeinheit kann motorisch fixierbar sein. Hierdurch kann auf einfache Weise eine durch den Benutzer gewählte Raumposition und Blickrichtung des Beobachtungsinstruments gehalten werden.

Allgemein kann das Drehen und Halten der Optikeinheit zur Einstellung der Orientierung der Stereobasis des Instruments manuell oder motorisch erfolgen. Sofern ein Motor die Einheit dreht und bei bestimmten Ausführungsformen auch in der eingestellten Position hält, kann diese Drehung auch automatisch erfolgen, in dem die Lage der Optikeinheit über einen Lagesensor erfasst wird. Ein Signal dieses Sensors kann von einer Steuerung verwendet werden, die Lage der Optikeinheit relativ zum tatsächlichen Horizont, zum Haltearm, zur Haltevorrichtung oder zum Haltebügel zu bestimmen und dann die Optikeinheit zu drehen, um eine gewünschte Ausrichtung der Stereobasis oder einen gewünschten Horizont bei Bewegung des Instruments zu halten. Die gewünschte Lage kann hierfür vom System automatisch bestimmt oder vorab oder während der Anwendung vom Anwender eingestellt werden.

Vorzugsweise ist der Haltearm motorisch verstellbar und/oder als robotischer Haltearm ausgebildet. Dabei kann es vorgesehen sein, dass der Haltearm über eine Bedienvorrichtung wie etwa einen Joystick oder auch über eine Datenverbindung mittels einer robotischen Steuerung bedient werden kann, wobei die robotische Steuerung programmierbar ausgestaltet sein kann, um die Optikeinheit in eine vorbestimmbare Position und/oder Orientierung zu bringen. Die zuvor genannte Drehung oder automatische Drehung der Optikeinheit und damit der Stereobasis kann alternativ oder zusätzlich durch Drehung von Teilen des robotischen Haltearms, an dem die Haltevorrichtung befestigt ist, erfolgen.

Weiter vorzugsweise ist am Haltebügel eine Bedieneinrichtung angeordnet oder in diesen integriert, die eine Mehrzahl von Bedientasten oder anderen Bedienelementen umfassen kann. So kann etwa eine Freigabetaste vorgesehen sein, die eine Fixierung des Haltearms löst und ein manuelles Positionieren ermöglicht. Dabei kann der Haltearm derart ausgestaltet und steuerbar sein, dass stets ein Gewicht des optischen Beobachtungsinstruments aufgenommen wird, so dass eine einfache manuelle Positionierung ermöglicht wird. Ferner kann beispielsweise durch Loslassen der Freigabetaste eine Arretierung bzw. eine Fixierung des Haltearms ausgelöst werden. Die Bedieneinrichtung kann weitere Bedienelemente umfassen, etwa zur Steuerung von Filtern der Optikeinheit und/oder zur Steuerung einer Beleuchtungsoptik und/oder zur Steuerung von Kamera- und Videofunktionen, wie Standbild oder speziellen Bildmodi zur Fluoreszenzbeobachtung. Die Tasten können in an sich bekannter Weise frei programmierbar sein, um an die Bedürfnisse des Anwenders angepasst zu werden.

Das optische Beobachtungsinstrument kann mit einer externen Steuerungseinrichtung verbindbar sein, welche zur Energieversorgung und Ansteuerung des mindestens einen elektronischen Bildaufnehmers sowie zur Anzeige des aufgenommenen Stereobilds oder zur Übertragung entsprechender Bildsignale an eine Anzeigeeinrichtung eingerichtet ist. Die Steuerungseinrichtung kann beispielsweise auch zur Versorgung und Steuerung einer Beleuchtungseinrichtung und/oder zur motorischen Bewegung der Haltevorrichtung ausgebildet sein.

Vorzugsweise umfasst das optische Beobachtungsinstrument oder die externe Steuerungseinrichtung eine elektronische Prozessoreinrichtung, die dazu eingerichtet ist, eine Spiegelung und/oder Vertauschung, in besonders bevorzugter Weise eine Spiegelung und eine Vertauschung, der von dem mindestens einen elektronischen Bildaufnehmer aufgenommenen Stereo-Halbbilder durchzuführen. Insbesondere in dem Fall, dass das optische Umlenkelement nur eine oder eine ungerade Anzahl von nacheinander im Strahlengang angeordneten reflektierenden Flächen aufweist, ist eine elektronisch durchgeführte Spiegelung der Halbbilder vorteilhaft. Hierdurch kann es ermöglicht werden, ein Stereobild zu erzeugen und für den Benutzer darzustellen, das dem Standort des Benutzers angepasst ist und einen vom Benutzer intuitiv erfassbaren räumlichen Eindruck des Objektfelds vermittelt.

Das optische Beobachtungsinstrument ist vorzugsweise zur Fluoreszenzbeobachtung, insbesondere zur stereoskopischen Fluoreszenzbeobachtung, ausgebildet und kann hierfür ein oder mehrere Filter, die wechselbar sein können, sowie beispielsweise vier elektronische Bildaufnehmer umfassen.

Ein erfindungsgemäßes optisches Beobachtungssystem umfasst ein optisches Beobachtungsinstrument und eine mit diesem verbundene Steuerungseinrichtung, die wie oben beschrieben ausgebildet sind.

Gemäß einem erfindungsgemäßen Verfahren zum Erzeugen eines Stereobilds eines Objektfelds wird die Optikeinheit eines optischen Beobachtungsinstruments, das wie oben beschrieben ausgebildet ist, in eine Raumposition gebracht. Hierfür kann ein Benutzer beispielsweise den Haltebügel erfassen und manuell durch Verstellung der Haltevorrichtung in die gewünschte Position bringen; es kann aber auch eine motorische Verstellung der Haltevorrichtung möglich sein. Zugleich kann dabei eine Blickrichtung zu einem zu beobachtenden Objektfeld bzw. eine Blickachse eingestellt werden. Durch Drehung der Optikeinheit um die erste Drehachse wird sodann eine Stereobasis entsprechend der Position des Benutzers ausgerichtet und die Stereo-Halbbilder aufgerichtet. Hierfür kann beispielsweise ein Gehäuse der Optikeinheit vom Benutzer ergriffen und manuell um die erste Drehachse gedreht werden; ggf. kann aber auch ein drehbares Bedienelement, das an dem Umlenkelement angeordnet ist, dessen Drehachse der Blickachse entspricht und das mit der Optikeinheit drehgekoppelt ist, vom Benutzer gedreht werden. Vorzugsweise wird mittels einer elektronischen Prozessoreinrichtung, die dem Beobachtungsinstrument oder einer externen Steuerungseinrichtung zugeordnet sein kann, eine elektronische Spiegelung und/oder Vertauschung der von dem mindestens einen elektronischen Bildaufnehmer aufgenommenen Stereo-Halbbilder durchgeführt. Die ggf. gespiegelten und/oder vertauschten Stereo-Halbbilder werden sodann auf einer Anzeigeeinrichtung, die zur stereoskopischen Darstellung geeignet ist, für den Benutzer dargestellt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung bevorzugter Ausführungsbeispiele und der beigefügten Zeichnung. Es zeigen:
Fig. 1 eine beispielhafte Prinzipskizze eines erfindungsgemäßen optischen Beobachtungsinstruments;
Fig. 2 ein erstes Ausführungsbeispiel eines erfindungsgemäßen optischen Beobachtungsinstruments in einer Seitenansicht;
Fig. 3 ein zweites Ausführungsbeispiel eines erfindungsgemäßen optischen Beobachtungsinstruments in einer Schrägansicht;
Fig. 4 ein drittes Ausführungsbeispiel eines erfindungsgemäßen optischen Beobachtungsinstruments in einer teilweise aufgeschnittenen Seitenansicht;
Fig. 5 das optische Beobachtungsinstrument gemäß Fig. 4 in einer weiteren Seitenansicht;
Fig. 6 eine Prinzipdarstellung des Verstellmechanismus des optischen Beobachtungsinstruments gemäß Fig. 4;
Fig. 7 eine teilweise transparente Teilansicht des Verstellmechanismus des optischen Beobachtungsinstruments gemäß Fig. 4;
Fig. 8 ein viertes Ausführungsbeispiel eines erfindungsgemäßen optischen Beobachtungsinstruments in einer Seitenansicht.

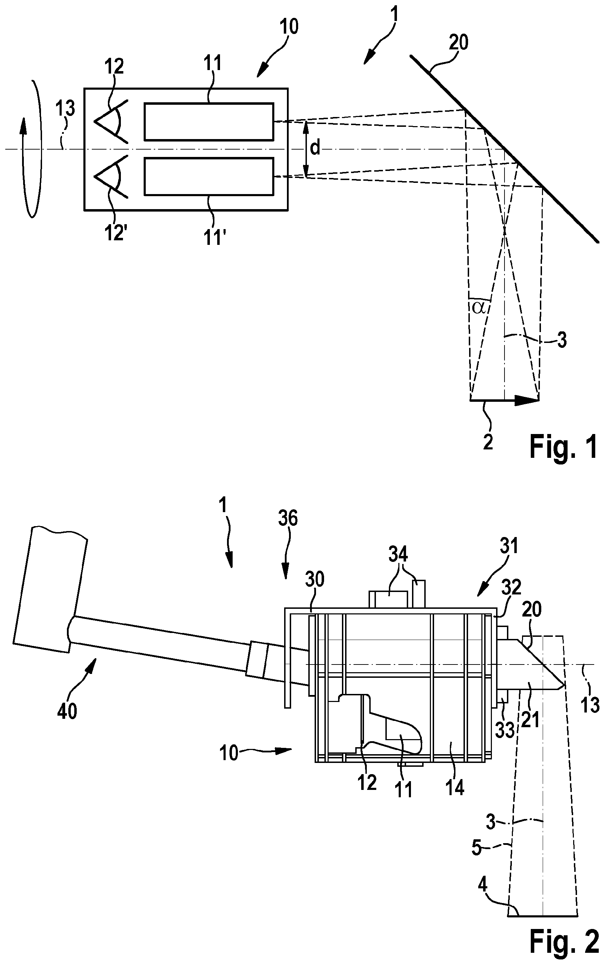

In Fig. 1 ist ein Ausführungsbeispiel eines erfindungsgemäßen optischen Beobachtungsinstruments in einer schematischen Prinzipdarstellung gezeigt. Das optische Beobachtungsinstrument 1 umfasst eine Optikeinheit 10, die zwei Stereokanäle 11, 11' aufweist, welche im dargestellten Beispiel jeweils ein Objektiv umfassen und denen jeweils ein elektronischer Bildaufnehmer 12, 12' zugeordnet ist. Die Stereokanäle 11, 11' können weitere optische Elemente umfassen. In Fig. 1 sind die Stereokanäle 11, 11' getrennt voneinander dargestellt; die Stereokanäle 11, 11' bzw. deren Objektive können jedoch auch gemeinsame optische Elemente umfassen, beispielsweise eine gemeinsame Frontlinse (s.u.). Die Objektive der Stereokanäle 11, 11' erzeugen jeweils ein Bild eines in einem Objektfeld angeordneten Objekts 2 auf dem jeweiligen Bildaufnehmer 12, 12', wobei in Fig. 1 die jeweiligen Strahlengänge nur teilweise dargestellt sind. Die Optikeinheit 10 ist um eine erste Drehachse 13 drehbar gelagert, welche einer Achse der Objektivanordnung der Optikeinheit 10 entspricht und die im gezeigten Beispiel eine Mittelachse zwischen den optischen Achsen der Objektive der Stereokanäle 11, 11' ist. Die Stereokanäle 11, 11' sind quer zur ersten Drehachse 13 gegeneinander versetzt, wobei der Versatz der optischen Achsen die Stereobasis d des Beobachtungsinstruments 1 darstellt.

Zur Umlenkung der aus Richtung des Objekts 2 einfallenden Strahlen in die Stereokanäle 11, 11' bzw. in die jeweiligen Objektive umfasst das optische Beobachtungsinstrument 1 einen als Planspiegel ausgebildeten Umlenkspiegel 20, an dem die Strahlen jeweils einmal reflektiert und zu den Objektiven der Stereokanäle 11, 11' umgelenkt werden. Die Blickrichtungen der beiden Stereokanäle 11, 11' unterscheiden sich um einen Stereowinkel α, der in Fig. 1 zwischen zwei Strahlen angedeutet ist, die von demselben Punkt des Objekts 2 ausgehen und von dem Umlenkspiegel 20 in die Objektive der Stereokanäle 11, 11' umgelenkt werden; die Blickrichtungen der Stereokanäle 11, 11' können aber auch parallel zueinander sein (nicht dargestellt). Der Umlenkspiegel 20 ist um 45° gegen die erste Drehachse 13 geneigt, wobei über die Fläche des Umlenkspiegels 20 die objektseitigen Strahlengänge beider Stereokanäle 11, 1 1' in unterschiedlichen Drehpositionen der Optikeinheit 10 in die jeweiligen Objektive umgelenkt werden. Die Blickachse 3 entspricht einer Winkelhalbierenden zwischen den objektseitigen Abschnitten der Strahlengänge der beiden Stereokanäle 11, 11' und ist um 90° gegenüber der ersten Drehachse 13 abgewinkelt. Die Blickrichtung des Beobachtungsinstruments 1 ist eine mittlere Blickrichtung zwischen den Blickrichtungen der Stereokanäle 11, 11', wobei die Blickachse 3 beispielsweise als durch den Umlenkspiegel 20 umgelenkte objektseitige Fortsetzung der Mittelachse zwischen den optischen Achsen der Objektive der Stereokanäle 11, 11' angesehen werden kann. Ein entlang der Blickachse 3 vom Objekt 2 auf den Umlenkspiegel 20 einfallender Strahl wird im gezeigten Beispiel in die Mittelachse, d.h. in die erste Drehachse 13, umgelenkt, wobei dieser Strahl jedoch nicht in einen der Stereokanäle 11, 11' gelangt. Durch Drehung der Optikeinheit 10 um die erste Drehachse 13 kann die Richtung der Stereobasis d verändert werden, um diese der Position eines Benutzers, der die von den Bildaufnehmern 12, 12' aufgenommenen Stereo-Halbbilder betrachtet, anzupassen und das angezeigte Stereobild entsprechend aufzurichten.

In Fig. 2 ist ein erstes Ausführungsbeispiel des erfindungsgemäßen optischen Beobachtungsinstruments in einer teilweise aufgeschnittenen Seitenansicht gezeigt. Gemäß dem ersten Ausführungsbeispiel, wie auch in den weiteren Ausführungsbeispielen, ist das Beobachtungsinstrument 1 ein Operationsmikroskop. Die Optikeinheit 10 entspricht dem Mikroskopkorpus des Operationsmikroskops. Die Optikeinheit 10 weist ein Optikgehäuse 14 auf, in dem optische und elektronische Bauelemente aufgenommen sind; in Fig. 2 sind optische Bauelemente eines Stereokanals 11 und ein elektronischer Bildaufnehmer 12 angedeutet.

Wie in Fig. 2 zu erkennen ist, ist der Mikroskopkorpus des Operationsmikroskops, d.h. die Optikeinheit 10, in einem Haltebügel 30 drehbar gehalten. Am objektseitigen Ende 31 des Haltebügels 30 weist dieser eine Platte 32 auf, an der die Optikeinheit 10 um die erste Drehachse 13 in einem Drehlager 33 drehbar gelagert ist. Dabei verlaufen die Strahlengänge der beiden Stereokanäle 11, 11' (s. Fig. 1) durch das Drehlager 33 hindurch. An dem dem objektseitigen Ende 31 entgegengesetzten objektfernen Ende 36 des Haltebügels 30 ist dieser mit einem Haltearm 40 verbunden, der aus mehreren zueinander abgewinkelten und gegebenenfalls zueinander verstellbaren Abschnitten besteht. Der Haltearm 40 kann beispielsweise als Stativ ausgebildet sein, das an einem Operationstisch befestigt werden kann oder auch unabhängig vom Operationstisch stehen kann, kann aber auch beispielsweise ein robotischer Haltearm sein. Der Haltebügel 30 kann dreh- und/oder schwenkbar mit dem Haltearm 40 verbunden sein. Durch den Haltearm 40 können Versorgungs- und Signalleitungen verlaufen, durch die die Optikeinheit 10 mit einer externen Steuerungseinrichtung verbunden ist. Der Haltebügel 30 kann Befestigungselemente 34 zum Befestigen eines nicht dargestellten Handgriffs aufweisen, um eine manuelle Verstellung des Haltearms 40 zur Wahl einer Position und Orientierung des Haltebügels 30 zu erleichtern.

An der Platte 32 ist weiter objektseitig der Umlenkspiegel 20 angeordnet, der im gezeigten Beispiel 45° schräg zur ersten Drehachse steht. Der Umlenkspiegel 20 ist in einem Spiegelgehäuse 21 gehalten, welches objektseitig des Lagers 33 angeordnet ist. Das Spiegelgehäuse 21 mit dem Umlenkspiegel 20 ist im gezeigten Beispiel um die erste Drehachse 13 drehbar an der Platte 32 angeordnet, kann aber auch drehfest mit der Platte 32 verbunden sein. In Fig. 2 sind die Blickachse 3, die die Blickrichtung des Operationsmikroskops definiert, das Objektfeld 4 und ein Kegel 5 angedeutet, der die objektseitigen Abschnitte der Strahlengänge der beiden Stereokanäle 11, 11' (s. Fig. 1) umfasst, einschließlich einer virtuellen Verlängerung über den Umlenkspiegel 20 hinaus.

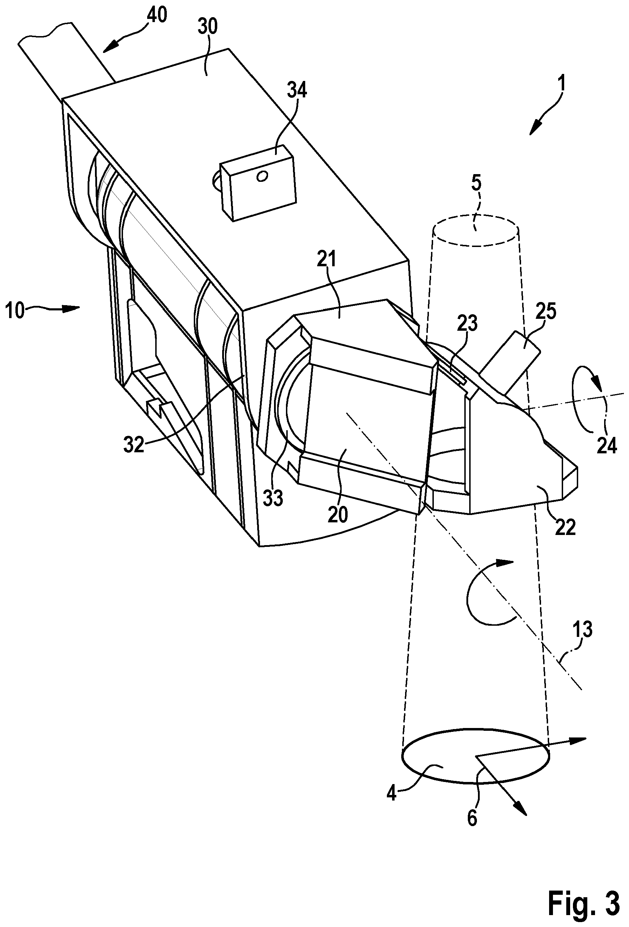

In Fig. 3 ist ein zweites Ausführungsbeispiel des erfindungsgemäßen optischen Beobachtungsinstruments in einer teilweise aufgeschnittenen Schrägansicht dargestellt. Gemäß diesem Ausführungsbeispiel weist das Beobachtungsinstrument 1 eine Optikeinheit 10, einen Haltebügel 30 und einen Haltearm 40 auf, welche wie zu Fig. 2 beschrieben ausgebildet sind. An der objektseitigen Platte 32 des Haltebügels 30 ist das Drehlager 33 zur drehbaren Lagerung der Optikeinheit 10 angeordnet, wobei objektseitig an dem Drehlager 33 ein erstes Spiegelgehäuse 21 mit dem ersten Umlenkspiegel 20 angeordnet ist.

Gemäß dem in Fig. 3 gezeigten zweiten Ausführungsbeispiel ist das Spiegelgehäuse 21 ebenfalls um die erste Drehachse 13 drehbar gelagert. Ferner ist auf der im Strahlengang objektseitigen Seite des ersten Spiegelgehäuses 21 ein zweites Spiegelgehäuse 22 mit einem zweiten Umlenkspiegel 23 angeordnet. Wie in Fig. 3 angedeutet ist, ist das zweite Spiegelgehäuse 22 an dem ersten Spiegelgehäuse 21 gehalten und um eine Schwenkachse 24, die senkrecht zur ersten Drehachse 13 steht, relativ zum ersten Spiegelgehäuse 21 drehbar. Am zweiten Spiegelgehäuse 22 ist ein Griffelement 25 zum manuellen Verstellen des ersten und des zweiten Umlenkspiegels 20, 23 vorgesehen.

Vom Objektfeld 4 innerhalb des Kegels 5 einfallende Strahlen werden vom zweiten Umlenkspiegel 23 näherungsweise in Richtung der Schwenkachse 24 zum ersten Umlenkspiegel 20 umgelenkt und dort näherungsweise in Richtung der ersten Drehachse 13 zur Optikeinheit 10. Die objektseitigen Abschnitte der Strahlengänge des ersten und des zweiten Stereokanals werden somit jeweils zweimal um etwa 90° umgelenkt. Durch Drehung des ersten Umlenkspiegels und des zweiten Umlenkspiegels 23 lässt sich die Blickrichtung des Beobachtungsinstruments 1 derart verändern, dass das beobachtbare Objektfeld 4 in zwei Richtungen verschoben werden kann (in Fig. 3 durch die Pfeile 6 angedeutet).

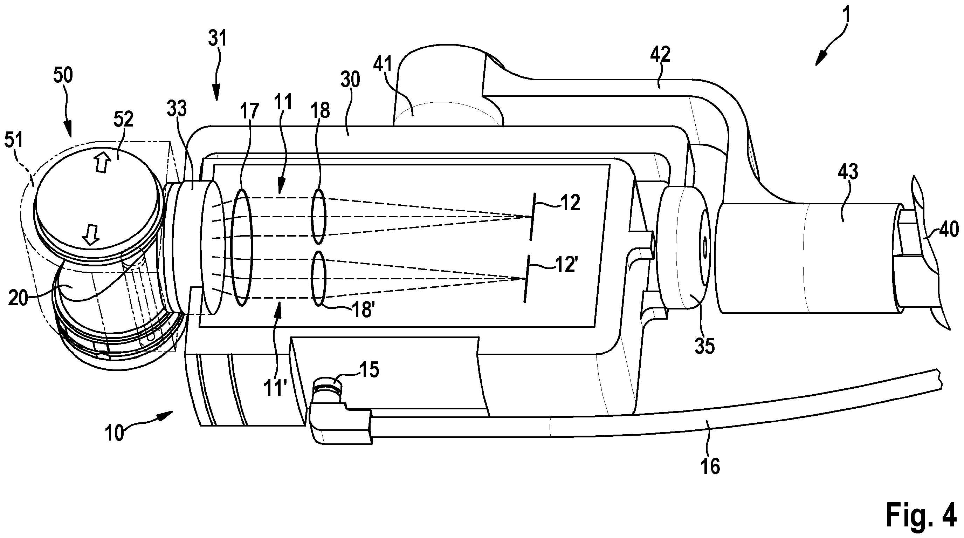

In den Figuren 4 bis 7 ist ein drittes Ausführungsbeispiel eines erfindungsgemäßen optischen Beobachtungsinstruments in unterschiedlichen Ansichten dargestellt.

Gemäß dem dritten Ausführungsbeispiel weist das optische Beobachtungsinstrument 1, welches ebenfalls ein Operationsmikroskop ist, eine Optikeinheit 10 auf, die wie zuvor beschrieben ausgestaltet ist, jedoch wie in Fig. 4 gezeigt einen seitlichen Anschluss 15 zum Anschließen eines Kabels 16 aufweist, über welches eine nicht näher dargestellte Beleuchtungseinrichtung der Optikeinheit 10 mit elektrischer oder optischer Energie versorgt werden kann. Das Kabel 16 kann ferner zur Verbindung mit einer externen Steuerungseinrichtung zur Energieversorgung, Steuerung und/oder Signalübertragung der elektronischen Bildaufnehmer sowie ggf. weiterer elektrischer oder elektronischer Komponenten der Optikeinheit 10 dienen. Nahe an dem Drehlager 33 ist eine Frontlinse 17 angeordnet, durch die die Strahlengänge des ersten und des zweiten Stereokanals 11, 11' (s. Fig. 1) verlaufen. Die Frontlinse 17 bildet ein gemeinsames optisches Element einer Objektivanordnung, die weitere optische Elemente umfasst, insbesondere die weiteren Objektivlinsen 18, 18', die in Fig. 4 symbolisch dargestellt sind und die jeweils einem der Stereokanäle 11, 11' zugeordnet sind. Die Objektivanordnung, die in Fig. 4 vereinfacht dargestellt ist, kann außer der Frontlinse 17 und den Objektivlinsen 18, 18' ggf. weitere Objektivlinsen und/oder optische Elemente umfassen. Die Objektivanordnung ist zum Erzeugen jeweils eines Bilds des Objektfelds auf den elektronischen Bildaufnehmern 12, 12' ausgebildet. Wie in Fig. 4 beispielhaft gezeigt ist, können die Strahlengänge der Stereokanäle 11, 11' nach dem Durchgang durch die Frontlinse 17 näherungsweise parallel zueinander verlaufen. Die Optikeinheit 10 kann weitere optische Bauteile umfassen wie etwa Filter, Umlenkelemente und/oder elektromechanische Bauteile, welche in Fig. 4 nicht gezeigt sind; auch das Gehäuse der Optikeinheit 10 ist in Fig. 4 nicht dargestellt. Die Objektivanordnung des ersten und des zweiten Ausführungsbeispiels kann in gleicher Weise wie bei dem dritten Ausführungsbeispiel aufgebaut sein.

Die Optikeinheit 10 ist an dem Haltebügel 30 mittels des Drehlagers 33 sowie des weiteren Drehlagers 35 drehbar gelagert. Der Haltebügel 30 ist nahezu mittig mittels eines Drehlagers 41 an einem Haltewinkel 42 drehbar gelagert, welcher Haltewinkel 42 mittels eines weiteren Drehlagers 43 an dem Haltearm 40 gelagert ist; zudem ist der Haltewinkel 42 längsverschiebbar am Haltearm 40 gehalten. Die Drehachsen des Drehlagers 41 und des weiteren Drehlagers 43 stehen senkrecht aufeinander und verlaufen näherungsweise durch den Schwerpunkt der am Haltearm angeordneten Komponenten, nämlich des Haltebügels 30, der Optikeinheit 10 und der nachfolgend beschriebenen Bedieneinheit 50. Die erste Drehachse, um die die Optikeinheit 10 im Haltebügel 30 drehbar gelagert ist, kann bei entsprechender Stellung des Drehlagers 41 mit der Drehachse des weiteren Drehlagers 43 fluchten.

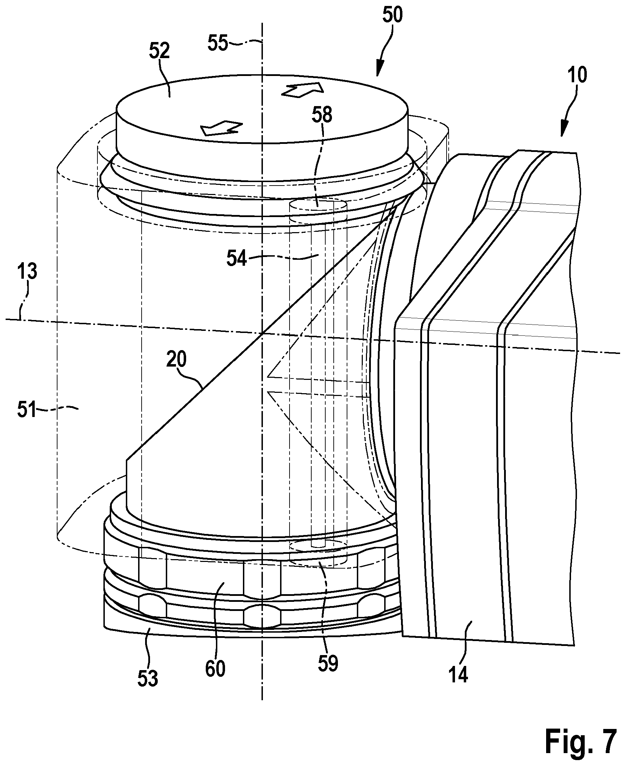

Die Bedieneinheit 50 umfasst ein Gehäuse 51, welches drehfest an der Platte 32 am objektseitigen Ende 31 des Haltebügels 30 im Bereich des Drehlagers 33 angeordnet ist; die Bedieneinheit kann aber auch um die erste Drehachse 13, um die die Optikeinheit 10 drehbar ist, drehbar am Haltebügel 30 gelagert sein. Innerhalb des Gehäuses 51 der Bedieneinheit 50 ist ein relativ zum Gehäuse 51 feststehender Umlenkspiegel 20 angeordnet, welcher als Planspiegel ausgebildet ist und der zur Umlenkung des ersten und des zweiten Strahlengangs vom Objektfeld zur Frontlinse 17 und in den ersten und den zweiten Stereokanal 11, 11' bzw. in die weiteren Objektivlinsen 18, 18' dient. An der dem Objektfeld entgegengesetzten Seite des Gehäuses 51 ist ein Drehrad 52 angeordnet, dessen Drehachse mit der Blickachse des Beobachtungsinstruments übereinstimmt. Wenn das Beobachtungsinstrument 1 oberhalb des zu beobachtenden Objektfelds positioniert ist, so ist das Drehrad 52 somit auf der Oberseite des Gehäuses 51 angeordnet. Wie unten näher beschrieben wird, kann die Optikeinheit 10 mittels des Drehrads 52 um die erste Drehachse 13 gedreht werden. An der objektseitigen Seite, d.h. im genannten Sinne auf der Unterseite, des Gehäuses 51 ist dieses durch ein transparentes Deckglas 53 abgeschlossen (s. Fig. 7). Das Deckglas 53 kann mittels einer Verbindungswelle 54, die eine Drehung des Drehrads 52 auf das Deckglas 53 überträgt, drehbar sein.

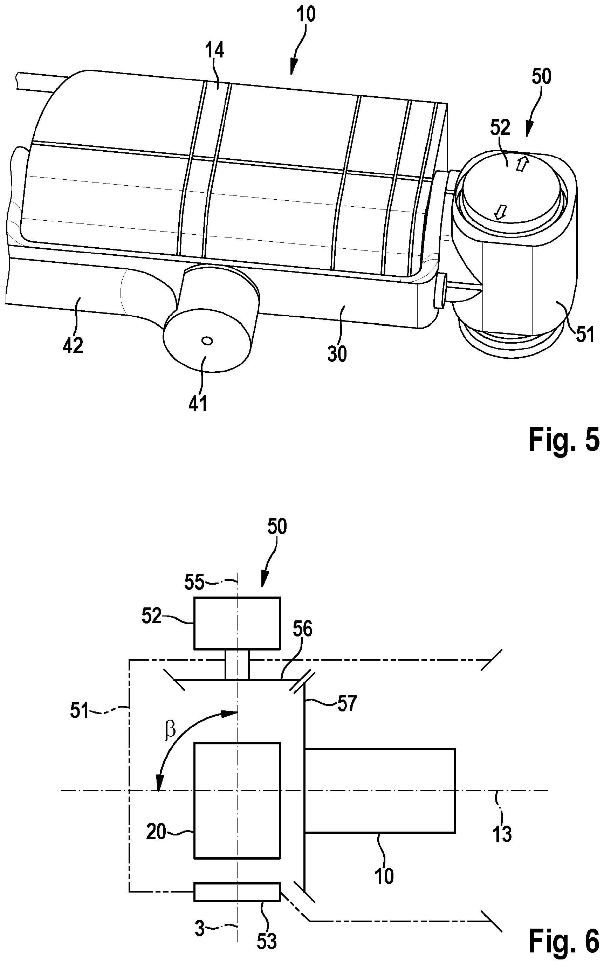

In Fig. 5 ist das optische Beobachtungsinstrument 1 gemäß dem dritten Ausführungsbeispiel in einer weiteren Seitenansicht gezeigt, wobei der Haltewinkel 42 mit dem Drehlager 41, an dem der Haltebügel 30 gelagert ist, erkennbar ist. Weiterhin sind die Optikeinheit 10 mit dem Optikgehäuse 14 und die Bedieneinheit 50 mit ihrem Gehäuse 51 und dem Drehrad 52 dargestellt.

In Fig. 6 ist das Getriebe, mittels dessen eine Drehung des Drehrads 52 zur Drehung der Optikeinheit 10 auf diese übertragen wird, in einer Prinzipdarstellung gezeigt. Wie in Fig. 6 symbolisch dargestellt ist, ist der Umlenkspiegel 20 feststehend in dem Gehäuse 51 der Bedieneinheit 50 aufgenommen. Das Drehrad 52 ist an der Oberseite des Gehäuses 51 angeordnet und um eine Drehachse 55 drehbar, die mit der Blickachse 3 des optischen Beobachtungsinstruments fluchtet. Auf der Drehachse 55 ist ein erstes Zahnrad 56 drehbar angeordnet und drehfest mit dem Drehrad 52 verbunden. Die Optikeinheit 10 ist innerhalb des nicht dargestellten Haltebügels 30 (s. Fig. 4, 5) um die erste Drehachse 13 drehbar gelagert. Auf der ersten Drehachse 13 ist ein zweites Zahnrad 57 angeordnet, das mit der Optikeinheit 10 drehfest verbunden ist. Das zweite Zahnrad 57 kämmt mit dem ersten Zahnrad 56, so dass eine Drehung des Drehrads 52 um dessen Drehachse 55 in eine Drehung der Optikeinheit 10 um die erste Drehachse 13 übertragen wird. Das erste und das zweite Zahnrad 56, 57 weisen die gleiche Anzahl Zähne und näherungsweise denselben Durchmesser auf, so dass eine Drehung des Drehrads 52 in eine gleich große Drehung der Optikeinheit 10 übertragen wird. Vorzugsweise sind das erste und das zweite Zahnrad 56, 57 als Kegelzahnräder ausgebildet. Das Gehäuse 51 der Bedieneinheit 50 kann wie Fig. 4, 5 und 7 gezeigt ausgestaltet sein, kann gemäß einer Variante des dritten Ausführungsbeispiels aber auch die Optikeinheit 10 umschließen und beispielsweise den Haltebügel 30 ersetzen.

Wie oben beschrieben, werden die Strahlengänge des ersten und des zweiten Stereokanals 11, 11' durch den Umlenkspiegel 20 zur Frontlinse 17 der Optikeinheit 10 umgelenkt und verlaufen durch das zweite Zahnrad 57. Im gezeigten Beispiel bildet die Blickachse 3 mit der ersten Drehachse 13 einen Winkel β = 90°, und der erste und der zweite Strahlengang, die von der Blickachse 3 bzw. der ersten Drehachse 13 jeweils um den halben Stereowinkel α abweichen können, werden an dem Umlenkspiegel jeweils um etwa 90° umgelenkt.

Wie in Fig. 7 gezeigt ist, kann die Bedieneinheit 50 eine parallel zur Drehachse 55 des Drehrads 52 verlaufende Verbindungswelle 54 aufweisen, die über ein Zahnrad 58 vom Drehrad 52 angetrieben wird und über ein weiteres Zahnrad 59 einen Drehring 60 antreibt, an dem das Deckglas 53 gehalten ist. Auf diese Weise kann bei einer Drehung des Drehrads 52 und der Optikeinheit 10 zugleich eine entsprechende, vorzugsweise gleich große Drehung des Deckglases 53 bewirkt werden.

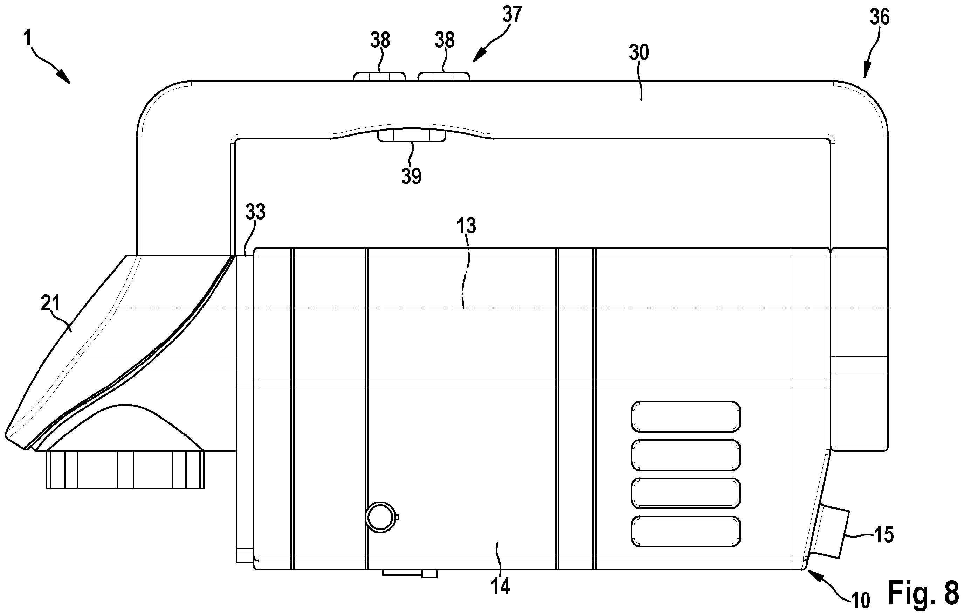

In Fig. 8 ist ein viertes Ausführungsbeispiel eines erfindungsgemäßen optischen Beobachtungsinstruments in einer Seitenansicht dargestellt. Bei diesem Ausführungsbeispiel ist der Haltebügel 30, in dem die Optikeinheit 10 drehbar gelagert ist, an seinem objektfernen Ende 36 fest mit einem nicht dargestellten robotischen Haltearm verbunden. Der robotische Haltearm ist derart ansteuerbar, dass er unabhängig von einer Position und Orientierung des optischen Beobachtungsinstruments 1 dessen Gewicht aufnimmt und eine eingestellte Position und Orientierung des Haltebügels 30 fixiert.

In den Haltebügel 30 ist eine Bedieneinrichtung 37 integriert, die oberseitig eine Mehrzahl von Bedientasten 38 zur Steuerung beispielsweise einer Lichtquelle oder von Filtern, die in der Optikeinheit 10 angeordnet sind, aufweist. Den Bedientasten 38 und insbesondere einer Fingerauflage zwischen den Bedientasten gegenüberliegend ist unterseitig eine Freigabetaste 39 angeordnet. Durch Drücken der Freigabetaste 39 wird die Fixierung der Position und Orientierung des Haltebügels 30 gelöst, wobei weiterhin das Gewicht des optischen Beobachtungsinstruments durch den robotischen Haltearm getragen wird; der Haltebügel 30 kann nun manuell in eine neue Position und Orientierung gebracht werden. Durch Loslassen der Freigabetaste 39 wird die Fixierung der Position und Orientierung des Haltebügels 30 wieder aktiviert. Zusätzlich oder alternativ kann die Freigabe den robotischen Haltearm betreffen, der durch Bedienung der Freigabetaste 39 in einen schwebend haltenden Zustand übergeht, in dem die Gelenke des Arms teilweise gelöst sind, jedoch das Gewicht des optischen Beobachtungsinstruments 1 halten, so dass dieses seine Position behält. Der Arm mit dem Instrument 1 kann dann vom Anwender frei bewegt werden. Durch Loslassen der Freigabetaste 39 wird der Haltearm wieder in der eingestellten Position verriegelt.

Zum Verändern der Drehposition der Optikeinheit 10 und somit zum Anpassen der Stereobasis bzw. des Horizonts kann das Gehäuse 14 der Optikeinheit 10 von dem Benutzer ergriffen und manuell gedreht werden. Eine eingestellte Drehposition wird durch einen nicht dargestellten Rastmechanismus, der an dem objektseitigen Drehlager 33 angeordnet ist, gehalten. Der Rastmechanismus umfasst beispielsweise eine in der Optikeinheit 10 gelagerte Sperrklinke, die federbelastet in Rastnuten eines fest mit dem Haltebügel 30 verbundenen Zahnrads eingreift; beim manuellen Drehen der Optikeinheit 10 wird die Federkraft überwunden und die Sperrklinke aus einer jeweiligen Rastnut ausgehoben, so dass die Optikeinheit 10 relativ zum Haltebügel 30 um die erste Drehachse 13 gedreht werden kann. Ausgehend von der in Fig. 8 dargestellten aufrechten Position kann die Optikeinheit 10 in beide Richtungen um jeweils 135° um die Drehachse 13 gedreht werden.

Der nicht dargestellte Umlenkspiegel ist fest in dem Spiegelgehäuse 21 angeordnet, das fest mit dem Haltebügel 30 verbunden ist bzw. eine einstückig mit diesem ausgebildete Oberseite haben kann. Das Gehäuse 14 der Optikeinheit weist weitere Bedienelemente sowie einen Anschluss 15 für ein Kabel auf. Im Übrigen ist das vierte Ausführungsbeispiel wie zuvor beschrieben, insbesondere entsprechend dem ersten Ausführungsbeispiel, ausgebildet.

Gemäß einem erfindungsgemäßen Verfahren wird die an dem Haltearm 40 bzw. dem Haltebügel 30 angeordnete Optikeinheit 10 von einem Benutzer in eine gewünschte Raumposition gebracht, beispielsweise manuell mittels eines am Haltebügel 30 angeordneten Handgriffs oder auch motorisch durch Steuerung entsprechender Aktuatoren mittels einer hierfür eingerichteten externen Steuerungseinrichtung. Hierdurch kann zugleich eine Blickrichtung bzw. eine Blickachse 3 zu einem zu beobachtenden Objektfeld 4 eingestellt werden; bei dem Ausführungsbeispiel gemäß Fig. 2 kann durch Drehung des Spiegelgehäuses 21 bzw. bei dem Ausführungsbeispiel gemäß Fig. 3 durch Drehung der Spiegelgehäuse 21, 22 die Blickrichtung zusätzlich in einem bzw. zwei Freiheitsgraden verändert werden. Bei den Ausführungsbeispielen gemäß Fig. 2 und Fig. 3 kann zum Einstellen der Richtung der Stereobasis sodann die Optikeinheit 10 vom Benutzer ergriffen und manuell um die erste Drehachse 13 gedreht werden. Bei dem Ausführungsbeispiel gemäß Fig. 4 bis 7 kann die Stereobasis durch eine Drehung des Drehrads 52, die eine entsprechende Drehung der Optikeinheit 10 bewirkt, manuell eingestellt werden. Mittels einer Prozessoreinrichtung, die beispielsweise in der Optikeinheit 10 oder in einer externen Steuerungseinrichtung angeordnet sein kann, kann eine elektronische Spiegelung der von den Bildaufnehmern 12, 12' aufgenommenen Stereo-Halbbilder vorgenommen werden. Zusätzlich kann auf elektronische Weise eine Vertauschung der von den Bildaufnehmern 12, 12' aufgenommenen Stereo-Halbbilder erfolgen. Die derart verarbeiteten Halbbilder werden sodann auf einer Anzeigeeinrichtung wie etwa einem für eine stereoskopische Darstellung eingerichteten Monitor dargestellt. Auf diese Weise kann ein Stereobild erzeugt und angezeigt werden, das dem Benutzer einen intuitiv erfassbaren räumlichen Eindruck des Objektfelds gibt.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Optisches Beobachtungsinstrument
- 2: Objekt
- 3: Blickachse
- 4: Objektfeld
- 5: Kegel
- 6: Pfeil
- 10: Optikeinheit
- 11, 11': Stereokanal
- 12, 12': Bildaufnehmer
- 13: Erste Drehachse
- 14: Gehäuse
- 15: Anschluss
- 16: Kabel
- 17: Frontlinse
- 18, 18': Objektivlinse
- 20: Umlenkspiegel
- 21: Spiegelgehäuse
- 22: Spiegelgehäuse
- 23: Umlenkspiegel
- 24: Schwenkachse
- 25: Griffelement
- 30: Haltebügel
- 31: Objektseitiges Ende
- 32: Platte
- 33: Drehlager
- 34: Befestigungselement
- 35: Drehlager
- 36: Objektfernes Ende
- 37: Bedieneinrichtung
- 38: Bedientasten
- 39: Freigabetaste
- 40: Haltearm
- 41: Drehlager
- 42: Haltewinkel
- 43: Drehlager
- 50: Bedieneinheit
- 51: Gehäuse
- 52: Drehrad
- 53: Deckglas
- 54: Verbindungswelle
- 55: Drehachse
- 56: Zahnrad
- 57: Zahnrad
- 58: Zahnrad
- 59: Zahnrad
- 60: Drehring

## Patentansprüche

1. Optisches Beobachtungsinstrument, insbesondere Operationsmikroskop oder Exoskop, umfassend eine Optikeinheit (10) mit einer Objektivanordnung und mindestens einem elektronischen Bildaufnehmer (12, 12'), wobei die Optikeinheit (10) einen ersten Stereokanal (11) mit einem ersten Strahlengang und einen zweiten Stereokanal (11') mit einem zweiten Strahlengang zum Aufnehmen eines Stereobilds eines Objektfelds (4) mit dem mindestens einen elektronischen Bildaufnehmer (12, 12') aufweist und wobei der erste und der zweite Strahlengang durch die Objektivanordnung verlaufen, und eine Haltevorrichtung, an der die Optikeinheit (10) um eine erste Drehachse (13) drehbar gelagert ist, die zumindest näherungsweise mit einer Achse der Objektivanordnung übereinstimmt, **dadurch gekennzeichnet, dass** das optische Beobachtungsinstrument (1) eine gegenüber der Achse der Objektivanordnung abgewinkelte Blickrichtung hat und ein objektseitig der Objektivanordnung angeordnetes Umlenkelement zur Umlenkung des ersten und des zweiten Strahlengangs in die Objektivanordnung umfasst, wobei die Optikeinheit (10) relativ zur Haltevorrichtung und relativ zum Umlenkelement um die erste Drehachse (13) drehbar gelagert ist.

2. Optisches Beobachtungsinstrument nach Anspruch 1, **dadurch gekennzeichnet, dass** die Blickrichtung näherungsweise um 90° gegenüber der Achse der Objektivanordnung abgewinkelt ist.

3. Optisches Beobachtungsinstrument nach Anspruch 1 bis 2, **dadurch gekennzeichnet, dass** die Optikeinheit (10) ein lang erstrecktes Optikgehäuse (14) aufweist, das sich zumindest näherungsweise in Richtung der Achse der Objektivanordnung erstreckt.

4. Optisches Beobachtungsinstrument nach einem der vorhergehenden Ansprüche, **dadurch gekennzeichnet, dass** die Objektivanordnung eine objektseitige Frontlinse (17) umfasst, wobei der erste und der zweite Strahlengang durch die Frontlinse (17) verlaufen.

5. Optisches Beobachtungsinstrument nach einem der vorhergehenden Ansprüche, **dadurch gekennzeichnet, dass** das optische Umlenkelement einen schräg zur Achse der Objektivanordnung stehenden Planspiegel umfasst.

6. Optisches Beobachtungsinstrument nach einem der vorhergehenden Ansprüche, **dadurch gekennzeichnet, dass** das optische Umlenkelement drehfest an der Haltevorrichtung angeordnet ist.

7. Optisches Beobachtungsinstrument nach einem der vorhergehenden Ansprüche, **dadurch gekennzeichnet, dass** die Optikeinheit (10) mit einem um eine zweite Drehachse (55) drehbaren Bedienelement drehgekoppelt ist, wobei die zweite Drehachse (55) zumindest näherungsweise parallel zur Blickrichtung gerichtet ist.

8. Optisches Beobachtungsinstrument nach Anspruch 7, **dadurch gekennzeichnet, dass** das drehbare Bedienelement auf einer dem Objektfeld (4) entgegengesetzten Seite an dem optischen Umlenkelement angeordnet ist.

9. Optisches Beobachtungsinstrument nach einem der vorhergehenden Ansprüche, **dadurch gekennzeichnet, dass** objektseitig des optischen Umlenkelements ein Deckglas (53) angeordnet ist.

10. Optisches Beobachtungsinstrument nach einem der vorhergehenden Ansprüche, **dadurch gekennzeichnet, dass** die Optikeinheit (10) eine Beleuchtungsoptik zur Beleuchtung des Objektfelds (4) umfasst mit einem Beleuchtungsstrahlengang, der durch die Objektivanordnung verläuft.

11. Optisches Beobachtungsinstrument nach einem der vorhergehenden Ansprüche, **dadurch gekennzeichnet, dass** die Haltevorrichtung einen Haltebügel (30) umfasst, der die Optikeinheit (10) übergreift und an dem die Optikeinheit (10) um die erste Drehachse (13) drehbar gelagert ist, wobei das optische Umlenkelement an einem objektseitigen Ende (31) des Haltebügels (30) gehalten ist.

12. Optisches Beobachtungsinstrument nach Anspruch 11, **dadurch gekennzeichnet, dass** die Haltevorrichtung einen Haltewinkel (42) und einen Haltearm (40) umfasst, wobei der Haltebügel (30) drehbar an dem Haltewinkel (42) angeordnet ist, welcher drehbar und/oder längsverschiebbar an dem Haltearm (40) angeordnet ist.

13. Optisches Beobachtungsinstrument nach Anspruch 11, **dadurch gekennzeichnet, dass** die Haltevorrichtung einen Haltearm (40) umfasst, wobei der Haltebügel (30) drehbar an dem Haltearm (40) angeordnet ist.

14. Optisches Beobachtungsinstrument nach einem der Ansprüche 11 bis 13, **dadurch gekennzeichnet, dass** die Haltevorrichtung derart ausgebildet ist, dass eine Position und/oder Orientierung der Optikeinheit (10) durch Reibschluss, Kraftschluss, Formschluss, elektromagnetisch und/oder motorisch fixierbar ist.

15. Verfahren zum Erzeugen eines Stereobilds eines Objektfelds, wobei die Optikeinheit (10) eines optischen Beobachtungsinstruments (1), das gemäß einem der vorhergehenden Ansprüche ausgebildet ist, in eine Raumposition gebracht und die Blickrichtung zu dem Objektfeld (4) eingestellt wird, durch Drehung der Optikeinheit (10) um die erste Drehachse (13) die Ausrichtung einer Stereobasis eingestellt wird und die von dem mindestens einen elektronischen Bildaufnehmer (12, 12') des optischen Beobachtungsinstruments (1) aufgenommenen Stereo-Halbbilder auf einer Anzeigeeinrichtung dargestellt werden.

## Claims

1. An optical observation instrument, in particular a surgical microscope or exoscope, comprising an optics unit (10) with an objective arrangement and at least one electronic image recorder (12, 12'), wherein the optics unit (10) has a first stereo channel (11) with a first beam path and a second stereo channel (11') with a second beam path for recording a stereo image of an object field (4) with the at least one electronic image recorder (12, 12') and wherein the first and the second beam path run through the objective arrangement, and a retaining device on which the optics unit (10) is mounted so as to be rotatable about a first axis of rotation (13) which at least approximately corresponds to an axis of the objective arrangement, **characterized in that**
the optical observation instrument (1) has a viewing direction angled with respect to the axis of the objective arrangement and comprises a deflection element arranged on the object-side of the objective arrangement for deflecting the first and the second beam path into the objective arrangement, wherein
the optics unit (10) is mounted so as to be rotatable about the first axis of rotation (13) relative to the retaining device and relative to the deflection element.

2. The optical observation instrument according to claim 1, **characterized in that** the viewing direction is angled at approximately 90° with respect to the axis of the objective arrangement.

3. The optical observation instrument according to claim 1 to 2, **characterized in that** the optics unit (10) has an elongated optics housing (14) which extends at least approximately in the direction of the axis of the objective arrangement.

4. The optical observation instrument according to one of the preceding claims, **characterized in that** the objective arrangement comprises an object-side front lens (17), wherein the first and the second beam path run through the front lens (17).

5. The optical observation instrument according to one of the preceding claims, **characterized in that** the optical deflection element comprises a plane mirror which is at an angle to the axis of the objective arrangement.

6. The optical observation instrument according to one of the preceding claims, **characterized in that** the optical deflection element is arranged in a rotationally-fixed manner on the retaining device.

7. The optical observation instrument according to one of the preceding claims, **characterized in that** the optics unit (10) is rotationally coupled to an operating element that is rotatable about a second axis of rotation (55), wherein the second axis of rotation (55) is directed at least approximately parallel to the viewing direction.

8. The optical observation instrument according to claim 7, **characterized in that** the rotatable operating element is arranged at the optical deflection element on a side opposite the object field (4).

9. The optical observation instrument according to one of the preceding claims, **characterized in that** a cover glass (53) is arranged on the object-side of the optical deflection element.

10. The optical observation instrument according to one of the preceding claims, **characterized in that** the optics unit (10) comprises illumination optics for illuminating the object field (4) with an illumination beam path which runs through the objective arrangement.

11. The optical observation instrument according to one of the preceding claims, **characterized in that** the retaining device comprises a retaining bracket (30) which engages over the optics unit (10) and on which the optics unit (10) is mounted so as to be rotatable about the first axis of rotation (13), wherein the optical deflection element is held at an object-side end (31) of the retaining bracket (30).

12. The optical observation instrument according to claim 11, **characterized in that** the retaining device comprises an angle bracket (42) and a retaining arm (40), wherein the retaining bracket (30) is arranged so as to be rotatable at the angle bracket (42), which is arranged so as to be rotatable and/or longitudinally displaceably at the retaining arm (40).

13. The optical observation instrument according to claim 11, **characterized in that** the retaining device comprises a retaining arm (40), wherein the retaining bracket (30) is arranged so as to be rotatable at the retaining arm (40).

14. The optical observation instrument according to one of claims 11 to 13, **characterized in that** the retaining device is designed in such manner that a position and/or orientation of the optics unit (10) can be fixed by frictional engagement, force-fitting engagement, form-fitting engagement, in an electromagnetic manner and/or in a motorized manner.

15. A method for generating a stereo image of an object field, wherein the optics unit (10) of an optical observation instrument (1) designed according to one of the preceding claims is brought into a spatial position and the viewing direction is set to the object field (4), the alignment of a stereo base is set by rotating the optics unit (10) about the first axis of rotation (13) and the stereo half images recorded by the at least one electronic image recorder (12, 12') of the optical observation instrument (1) are displayed on a display device.

## Revendications

1. Instrument d'observation optique, notamment microscope chirurgical ou exoscope, comprenant une unité optique (10) comportant un agencement d'objectifs et au moins un enregistreur d'image électronique (12, 12'), dans lequel l'unité optique (10) a un premier canal stéréo (11) avec un premier trajet de faisceau et un second canal stéréo (11') avec un second trajet de faisceau pour enregistrer une image stéréo d'un champ objet (4) avec l'au moins un enregistreur d'image électronique (12, 12') et dans lequel le premier et le second trajet de faisceau traversent l'agencement d'objectifs, et un dispositif de maintien, sur lequel l'unité optique (10) est montée rotative autour d'un premier axe de rotation (13), qui coïncide au moins approximativement avec un axe de l'agencement d'objectifs, **caractérisé en ce que**
l'instrument d'observation optique (1) a une direction de visée qui est angulaire par rapport à l'axe de l'agencement d'objectifs et comprend un élément de déviation agencé sur le côté objet de l'agencement d'objectifs pour dévier les premier et second trajets de faisceau dans l'agencement d'objectifs, dans lequel l'unité optique (10) est montée rotative autour du premier axe de rotation (13) par rapport au dispositif de maintien et par rapport à l'élément de déviation.

2. Instrument d'observation optique selon la revendication 1, **caractérisé en ce que** la direction de visée est angulaire d'environ 90° par rapport à l'axe de l'agencement d'objectifs.

3. Instrument d'observation optique selon la revendication 1 à 2, **caractérisé en ce que** l'unité optique (10) présente un boîtier optique allongé (14), qui s'étend au moins approximativement dans la direction de l'axe de l'agencement d'objectifs.

4. Instrument d'observation optique selon l'une des revendications précédentes, **caractérisé en ce que** l'agencement d'objectifs comprend une lentille frontale (17) côté objet, dans lequel les premier et second trajets de faisceau traversent la lentille frontale (17).

5. Instrument d'observation optique selon l'une des revendications précédentes, **caractérisé en ce que** l'élément de déviation optique comprend un miroir plan s'étendant obliquement par rapport à l'axe de l'agencement d'objectifs.

6. Instrument d'observation optique selon l'une des revendications précédentes, **caractérisé en ce que** l'élément de déviation optique est agencé de manière fixe en rotation sur le dispositif de maintien.

7. Instrument d'observation optique selon l'une des revendications précédentes, **caractérisé en ce que** l'unité optique (10) est accouplée en rotation à un élément de commande qui peut être tourné autour d'un second axe de rotation (55), dans lequel le second axe de rotation (55) est dirigé au moins approximativement parallèlement à la direction de visée.

8. Instrument d'observation optique selon la revendication 7, **caractérisé en ce que** l'élément de commande rotatif est agencé sur l'élément de déviation optique sur un côté opposé au champ objet (4).

9. Instrument d'observation optique selon l'une des revendications précédentes, **caractérisé en ce qu'**un verre de protection (53) est agencé sur le côté objet de l'élément de déviation optique.

10. Instrument d'observation optique selon l'une des revendications précédentes, **caractérisé en ce que** l'unité optique (10) comprend une optique d'éclairage pour l'éclairage du champ objet (4) avec un trajet de faisceau d'éclairage, qui traverse l'agencement d'objectifs.

11. Instrument d'observation optique selon l'une des revendications précédentes, **caractérisé en ce que** le dispositif de maintien comprend un étrier de maintien (30), qui chevauche l'unité optique (10) et sur lequel l'unité optique (10) est montée rotative autour du premier axe de rotation (13), dans lequel l'élément de déviation optique est maintenu à une extrémité côté objet (31) de l'étrier de maintien (30).

12. Instrument d'observation optique selon la revendication 11, **caractérisé en ce que** le dispositif de maintien comprend une équerre de maintien (42) et un bras de maintien (40), dans lequel l'étrier de maintien (30) est agencé rotatif sur l'équerre de maintien (42), qui est agencée rotative et/ou déplaçable longitudinalement sur le bras de maintien (40).

13. Instrument d'observation optique selon la revendication 11, **caractérisé en ce que** le dispositif de maintien comprend un bras de maintien (40), dans lequel l'étrier de maintien (30) est agencé rotatif sur le bras de maintien (40).

14. Instrument d'observation optique selon l'une quelconque des revendications 11 à 13, **caractérisé en ce que** le dispositif de maintien est conçu de telle manière qu'une position et/ou une orientation de l'unité optique (10) peut être fixée par friction, accouplement de force, ajustement de forme, par voie électromagnétique et/ou par moteur.

15. Procédé de génération d'une image stéréo d'un champ objet, dans lequel l'unité optique (10) d'un instrument d'observation optique (1), qui est conçu selon l'une quelconque des revendications précédentes, est amené dans une position spatiale et la direction de visée est ajustée au champ objet (4), l'alignement d'une base stéréo est ajusté en faisant tourner l'unité optique (10) autour du premier axe de rotation (13) et les demi-images stéréo enregistrées par le au moins un enregistreur d'image électronique (12, 12') de l'instrument d'observation optique (1) sont représentées sur un dispositif d'affichage.
